# EUROPEAN PATENT APPLICATION

(11) **EP 2 351 748 A1**
(43) Date of publication of application: **03.08.2011**
(21) Application number: 09819245.3
(22) Date of filing: 08.10.2009
(51) Int. Cl.: C07D 401/04, A61K 31/4725, A61P 1/04, A61P 1/16, A61P 3/00, A61P 9/00, A61P 9/12, A61P 11/00, A61P 11/06, A61P 13/10, A61P 17/00, A61P 19/02, A61P 19/10, A61P 25/00, A61P 25/14, A61P 25/16, A61P 25/18, A61P 25/24, A61P 25/28, A61P 27/14

(54) **ISOQUINOLINE DERIVATIVE, AND PDE INHIBITOR COMPRISING SAME AS ACTIVE INGREDIENT**

(30) Priority: 09.10.2008 JP 2008262340
(71) Applicant: Kyorin Pharmaceutical Co., Ltd., Tokyo 101-8311 (JP)
(72) Inventor: KOHNO Yasushi, Shimotsuga-gun Tochigi 329-0114 (JP); TAKITA Satoshi, Shimotsuga-gun Tochigi 329-0114 (JP); KOJIMA Akihiko, Shimotsuga-gun Tochigi 329-0114 (JP)
(74) Representative: Weinberger, Rudolf
(86) International application number: PCT/JP2009/067546
(87) International publication number: WO 2010/041711

(57) **Abstract**

The present invention provides a novel isoquinoline derivative which is useful as a pharmaceutical agent having a phosphodiesterase inhibitory activity.

The isoquinoline derivative of the present invention is represented by the following general formula (1):

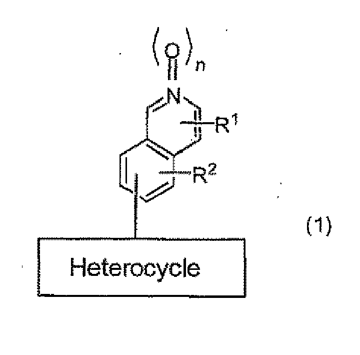

## Description

### Technical Field

The present invention relates to an isoquinoline derivative, or a salt or hydrate thereof, which is useful as a phosphodiesterase (PDE) inhibitor.

### Background Art

Phosphodiesterase (PDE) is an enzyme for decomposing cyclic AMP (cAMP) and cyclic GMP (cGMP) which are secondary messengers present in the living bodies. Up to now, 1 to 11 type PDEs have been found. Which of cAMP or cGMP is decomposed or both which are decomposed is different depending on the types of PDEs. There is observed difference in the kind of tissues in which PDEs are distributed. It has been considered that the cellular reactions are controlled by various types of PDEs depending on the kinds of internal organs.

Up to now, there have been developed and known a number of PDE inhibitors. For instance, the PDE3 inhibitor has been expected to be an agent for the treatment of, for instance, angina, heart failure and hypertension or a platelet aggregation inhibitor or an antasthmatic agent; and the PDE4 inhibitor has been expected to be an agent for treating, for instance, bronchial asthma, chronic obstructive pulmonary diseases (COPD), interstitial pneumonia, interstitial cystitis, allergic conjunctivitis, allergic rhinitis, atopic dermatitis, osteoporosis, osteoarthritis of knee, rheumatoid arthritis, non-alcoholic fatty liver, multiple sclerosis, Crohn's disease, inflammatory colitis, Alzheimer's disease, dementia. Parkinson's disease, and depression.
The PDE5 inhibitor has already been clinically used as an agent for the treatment of the male impotence. Moreover, there has recently been reported that minocycline is active as a PDE10A modulator after it was tried in patients suffering from Huntington' s disease (Patent Document 1). There have also publicly been opened patent publications which disclose that the PDE10 inhibitor is effective as an agent for the treatment of a variety of mental disorders such as Huntington's disease, Alzheimer's disease, dementia, Parkinson's disease, and schizophrenia (Patent Document 2). However, the compound disclosed in Patent Document 1 is related to a compound having, as the basic skeleton, an indoledione, while the compound disclosed in Patent Document 2 is related to a compound in which the group directly linked to the isoquinoline ring is not a hetero ring, but a phenyl group. In addition, the pamphlet of an international publication was published in the latest day, which shows that it is also effective for adiposis and metabolic syndromes (see Patent Document 3). However, the compound disclosed in Patent Document 3 is related to the compound in which the hetero ring is linked to the isoquinoline ring through a C-N bond.

There has been reported a compound having a hetero bicyclic structure as a PDE inhibitor (Patent Document 4). In the compound disclosed in Patent Document 4, however, the substituent at the 2-position locatede between neighboring two nitrogen atoms on the quinazoline ring of the compound is an amino group or a nitrogen atom-containing group and therefore, the compound is different, in the structure, from that of the present invention. In addition, there have been reported hetero bicyclic quinolone derivatives (Patent Documents 5 to 7) and hetero bicyclic quinazoline derivatives, hetero bicyclic benzoxazinone derivatives or the like (Patent Documents 8 to 11), although these compounds are not PDE inhibitors. However, in the compounds disclosed in Patent Documents 5 and 6 differ, in the substituents on the Het ring, from the compound according to the present invention. Moreover, the compound disclosed in Patent Document 7 has a phosphorus atom-containing group located at the 3-position on a quinolone ring and therefore, it is different from the compound of the present invention. Furthermore, even if the basic skeleton of the compound disclosed in Patent Document 8 and represented by the formula I corresponds to the heterocycle of the compound of the present invention, the position, at which a hetero ring corresponding to the heterocycle is linked to the basic skeleton of the formula I, is different from that of the compound according to the present invention. The compound disclosed in Patent Document 9 is linked to a heteroaryl group through a nitrogen atom-containing ring and accordingly, it differs from the compound of the present invention. In the compound disclosed in Patent Document 10, even if the basic skeleton (ZZ') of the compound represented by the formula I is a quinoline ring, a quinolinone ring or a quinazoline ring, the substituent R3 located on each of these rings differs, in the position on the ring and the kind of the substituent, from that of the compound according to the present invention. In the compound disclosed in Patent Document 11, even if the basic skeleton of the compound corresponds to the heterocycle of the compound of the present invention, it is different from the compound of the present invention at a position at which the basic skeleton represented by the formula I is linked to the hetero ring corresponding to the heterocycle of the compound of the present invention.

### Prior Art References:

### Patent Documents:

Patent Document 1: WO 01/024781 Pamphlet;
Patent Document 2: JP-A-2002-363103;
Patent Document 3: WO 2005/120514 Pamphlet;
Patent Document 4: WO 2005/087749 Pamphlet;
Patent Document 5: JP-A-63-280078;
Patent Document 6: EP 0290153;
Patent Document 7: JP-A-2001-278890;
Patent Document 8: WO 2006/015259 Pamphlet;
Patent Document 9: WO 2002/020488 Pamphlet;
Patent Document 10: WO 2007/076092 Pamphlet;
Patent Document 11: WO 2006/039718 Pamphlet

### Summary of the Invention:

### Subject to be Attained by the Invention:

It is an object of the present invention to provide an isoquinoline derivative which has an excellent phosphodiesterase-inhibitory action and which shows almost no side effect.

### Means for Attaining the Subject:

The inventors of this invention have conducted intensive studies to develop a compound which has a phosphodiesterase-inhibitory activity and which is highly safe, and as a result, have found that a novel isoquinoline derivative having a structure different from those of the conventionally known PDE inhibitors shows a PDE-inhibitory action and have thus completed the present invention.

More specifically, the present invention relates to the following inventions: 1) An isoquinoline derivative represented by the following general formula (1), an optically active derivative thereof or a pharmaceutically acceptable salt or hydrate thereof:

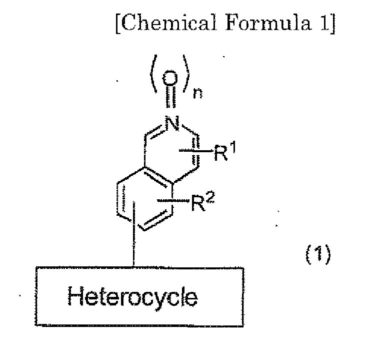

[wherein R¹ and R² may be the same or different and each represents a hydrogen atom or a halogen atom;
n represents 0 or 1; and
Heterocycle represents a heterocyclic group represented by one of the following general formulas:

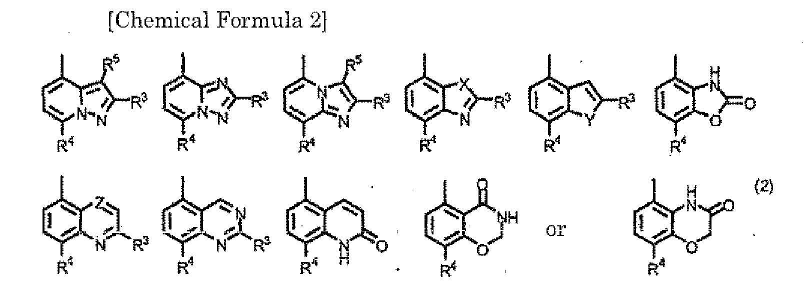

[wherein R³ represents a hydrogen atom, an alkyl group having 1 to 6 carbon atoms which may be substituted with a halogen atom, or a cycloalkyl group having 3 to 8 carbon atoms; R⁴ represents an alkoxy group having 1 to 6 carbon atoms, an amino group or an alkylamino group having 1 to 6 carbon atoms; R⁵ represents a hydrogen atom or a halogen atom; X represents NH, O or S; Y represents O or S; and Z represents CH or N]. 2) The isoquinoline derivative represented by the foregoing general formula (1), an optically active derivative thereof or a pharmaceutically acceptable salt or hydrate thereof, as set forth in the foregoing item 1), wherein the position of the Heterocycle moiety linked to the isoquinoline ring is represented by the following general formula (la):

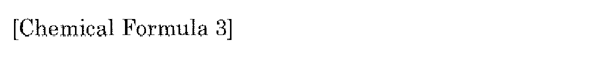

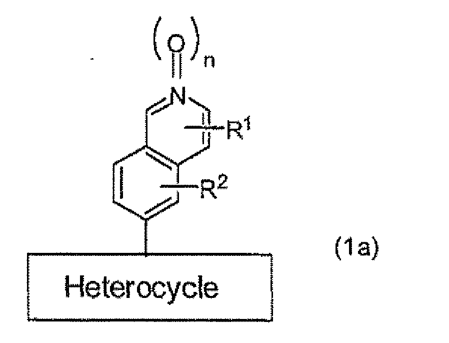

[wherein R¹, R², n and Heterocycle are the same as those defined above]. 3) The isoquinoline derivative represented by the foregoing general formula (1), an optically active derivative thereof or a pharmaceutically acceptable salt or hydrate thereof, as set forth in the foregoing item 1), wherein the compound represented by the foregoing general formula (1) is one represented by the following general formula:

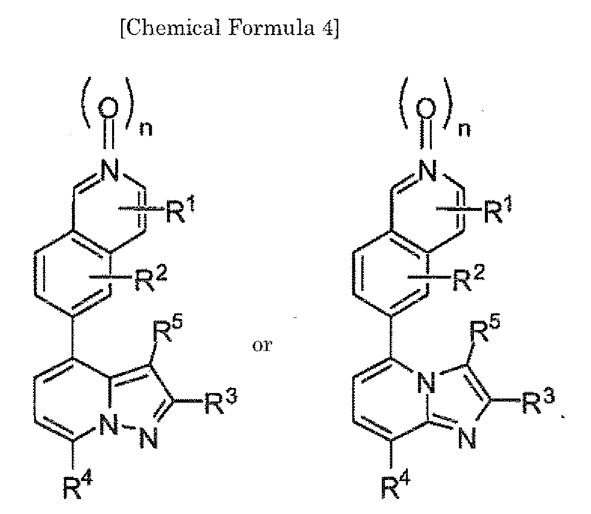

[wherein R¹, R², R³, R⁴, R⁵ and n are the same as those defined above].
4) The isoquinoline derivative represented by the foregoing general formula (1), an optically active derivative thereof or a pharmaceutically acceptable salt or hydrate thereof, as set forth in any one of the foregoing items 1) to 3), wherein the substituent R⁴ of the compound represented by the foregoing general formula (1) is an alkoxy group having 1 to 6 carbon atoms.
5) The isoquinoline derivative represented by the foregoing general formula (1), an optically active derivative thereof or a pharmaceutically acceptable salt or hydrate thereof, as set forth in any one of the foregoing items 1) to 3), wherein the substituent R⁴ of the compound represented by the foregoing general formula (1) is an alkylamino group having 1 to 6 carbon atoms.
6) The isoquinoline derivative represented by the foregoing general formula (I), an optically active derivative thereof or a pharmaceutically acceptable salt or hydrate thereof, as set forth in the foregoing item 1), wherein the compound represented by the foregoing general formula (1) is:
   (1) 6-(7-Methoxy-2-trifluoromethylpyrazolo[1,5-a]pyridin-4-yl)isoquinoline;
   (2) 5-Chloro-6-(7-methoxy-2-trifluoromethylpyrazolo[1,5-a]pyridin-4-yl)iso quinoline;
   (3) 6-(3-Chloro-7-methoxy-2-trifluoromethylpyrazolo[1,5-a] pyridin-4-yl)iso quinoline;
   (4) 3-Chloro-4-(isoquinolin-6-yl)-N-methyl-2-trifluoromethylpyrazalo[1,5-a] pyridine-7-amine;
   (5) 6-(3-Chloro-8-methoxy-2-trifluoromethylimidazo[1,2-a]pyridin-5-yl)iso quinoline; or
   (6) 3-Chloro-5-(isoquinolin-6-yl)-N-methyl-2-trifluoromethylimidazo[1,2-a] pyridine-8-amine.
   (7) A phosphodiesterase (PDE) inhibitor comprising, as an effective component, an isoquinoline derivative, an optically active derivative thereof or a pharmaceutically acceptable salt or hydrate thereof, as set forth in any one of the foregoing items 1) to 6).
   (8) A pharmaceutical composition comprising, as an effective component, an isoquinoline derivative, an optically active derivative thereof or a pharmaceutically acceptable salt or hydrate thereof, as set forth in any one of the foregoing items 1) to 6).
   (9) The pharmaceutical agent as set forth in the foregoing item 8) for preventing or treating angina, heart failure, hypertension, bronchial asthma, chronic obstructive pulmonary diseases (COPD), interstitial pneumonia, interstitial cystitis, allergic conjunctivitis, allergic rhinitis, atopic dermatitis, osteoporosis, rheumatoid arthritis, osteoarthrites of knee, non-alcoholic fatty liver, multiple sclerosis, Crohn's disease, inflammatory colitis, Huntington's disease, Alzheimer's disease, dementia, Parkinson's disease, depression, schizophrenia, obesity and metabolic syndromes.

### Effects of the Invention:

According to the present invention, it has been found that a novel isoquinoline derivative and an addition salt thereof show excellent PDE-inhibitory action. Such a compound having a PDE-inhibitory action is useful as an agent for treating angina, heart failure and hypertension; a platelet aggregation-inhibitory agent; or an agent for preventing or treating bronchial asthma, chronic obstructive pulmonary diseases (COPD), interstitial pneumonia, interstitial cystitis, allergic conjunctivitis, allergic rhinitis, atopic dermatitis, osteoporosis, rheumatoid arthritis, osteoarthritis of knee, non-alcoholic fatty liver, multiple sclerosis, Crohn's disease, inflammatory colitis, a variety of mental disorders such as Huntington's disease, Alzheimer's disease, dementia, Parkinson's disease, depression and schizophrenia, obesity and metabolic syndromes as well as an agent for the treatment of the male impotence.

### Mode for Carrying out the Invention:

In the present invention, the term "alkyl group having 1 to 6 carbon atoms" means a linear or branched alkyl group having 1 to 6 carbon atoms and it preferably represents an alkyl group having 1 to 4 carbon atoms. Specific examples thereof include methyl group, ethyl group, propyl group, isopropyl group, butyl group, isobutyl group, sec-butyl group, tert-butyl group, and the like.

The term "alkyl group which may be substituted with a halogen atom" means an alkyl group having 1 to 6 carbon atoms, which is unsubstituted or substituted with a halogen atom, preferably an alkyl group having 1 to 6 carbon atoms, in which all of the hydrogen atoms are substituted with fluorine atoms, more preferably an alkyl group having 1 to 4 carbon atoms, in which all of the hydrogen atoms are substituted with fluorine atoms, and particularly preferably a trifluoromethyl group.

The term "cycloalkyl group having 3 to 8 carbon atoms" means, for instance, cyclopropyl group, cyclobutyl group, cyclopentyl group, cyclohexyl group, cycloheptyl group, and cyclooctyl group, with cyclopropyl group being preferably used.
The term "alkoxy group having 1 to 6 carbon atoms" means a linear or branched alkoxy group having 1 to 6 carbon atoms and preferably an alkoxy group having 1 to 4 carbon atoms. Specific examples thereof are methoxy group, ethoxy group, propoxyl group, isopropoxy group, butoxy group, isobutoxy group, sec-butoxy group, tert-butoxy group, and the like.
The term "alkylamino group having 1 to 6 carbon atoms" means a linear or branched alkylamino group having 1 to 6 carbon atoms and preferably an alkylamino group having 1 to 4 carbon atoms. Specific examples thereof include methylamino group, ethylamino group, propylamino group, isopropylamino group, butylamino group, isobutylamino group, sec-butylamino group, tert-butylamino group, dimethylamino group, methyl(ethyl)amino group and the like.
The term "halogen atom" means fluorine, chlorine, bromine and iodine atoms.
As the pharmaceutically acceptable salts of the isoquinoline derivative, there may be listed, for instance, acid-addition salts such as hydrochloride, hydrobromide, acetate, trifluoroacetate, methanesulfonate, citrate, tartarate and the like.
The compound of the present invention represented by Formula (1) can be prepared according to, for instance, the synthetic route scheme A defined below:

### [Synthetic route scheme A]

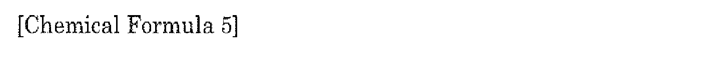

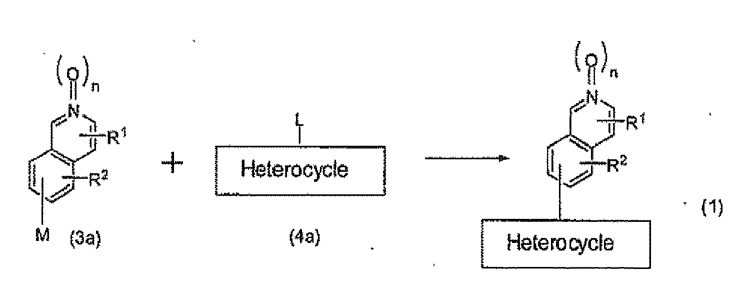

In the synthetic route scheme A, the compound of Formula (1) can be prepared by subjecting, to the Stille cross-coupling reaction or the Suzuki-Miyaura cross-coupling reaction, a compound represented by the following general formula (3a) and a compound represented by the following general formula (4a):

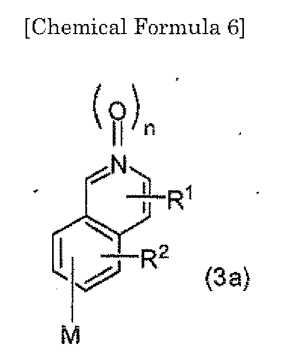

[wherein M represents -SnBus, -SnMes, -B(OH)₂, -B(OMe)₂ or a group represented by the following formula:

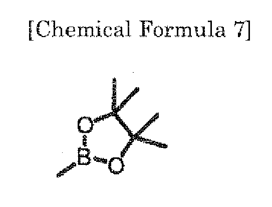

and R¹, R², and n are the same as those defined above];

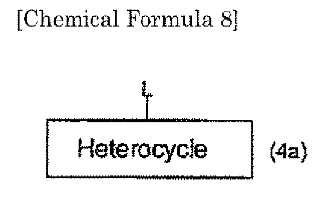

[wherein L represents a halogen atom or a trifluoromethanesulfonyloxy group and Heterocycle is the same as that defined above].

In case of the Stille coupling using a tin compound as a substrate, the reaction can be carried out at a temperature from ordinary temperature to the refluxing temperature, while using a solvent such as toluene, N-methylpyrrolidone, dimethylformamide (DMF), tetrahydrofuran (THF), 1,4-dioxane and the like, if necessary in the presence of a base such as diisopropylamine, using a catalyst, a Pd(0) complex such as tetrakis(triphenylphosphine) palladium [Pd(PPh₃)₄] and the like. Alternatively, the compound of Formula (1) can be also prepared according to a method in which a ligand as an organic phosphorus atom-containing compound such as triphenylphosphine (PPh₃), 1,1-bis(diphenylphosphino)ferrocene (dppf) and the like is added to a Pd(0) complex such as bis(dibenzylideneacetone) palladium (0) [Pd(dba)2] and the like; or a method which makes use of a Pd(II) complex such as palladium acetate [Pd(OAc)₂], bis(acetonitrile) dichloropalladium (II) [PdCl₂(MeCN)₂], dichlorobis(triphenylphosphine) palladium (II) [PdCl₂(PPh₃)₂], [1,1'-bis(diphenylphosphino)ferrocene]dichloropalladium (II) [PdCl₂(dppf)] and the like. In case of the Suzuki-Miyaura cross-coupling reaction using an organic boron compound, the reaction can be carried out at a temperature ranging from ordinary temperature to the refluxing temperature in the presence of a base such as potassium carbonate, cesium carbonate, sodium hydroxide, potassium hydroxide, potassium phosphate or diisopropylamine, while using, as a solvent, dimethoxyethane, DMF, THF, acetonitrile or 1,4-dioxane and using a palladium compound such as Pd(PPh₃)₄ as a catalyst. In this respect, it is also possible to use, as such a catalyst, those used in the Stille cross-coupling reaction, in addition to Pd(PPh₃)₄.

Moreover, the compound represented by Formula (1) can also be prepared according to the following synthetic route scheme B:

### [Synthetic Route Scheme B]

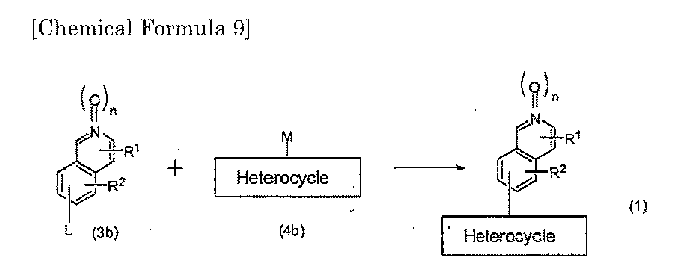

In the synthetic route scheme B, the compound of Formula (1) can be prepared by subjecting, to the Stille cross-coupling reaction or the Suzuki-Miyaura cross-coupling reaction, a compound represented by the following general formula (3b) and a compound represented by the following general formula (4b):

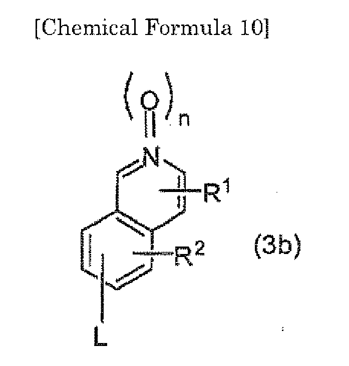

[wherein R¹, R², n and L are the same as those defined above]; and

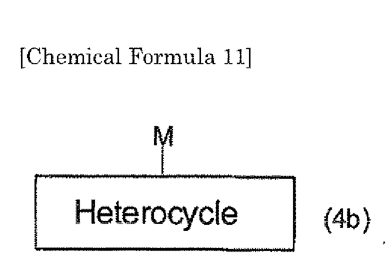

[wherein Heterocycle and M are the same as those defined above].
The reaction can be carried out by the same method used in the synthetic route scheme A.

In the synthetic route schemes A and B, the compounds represented by the general formulas (4a) and (4b), in which Heterocycle represents a pyrazolopyridine can be prepared according to the following synthetic route scheme C or C':

### [Synthetic Route Scheme C]

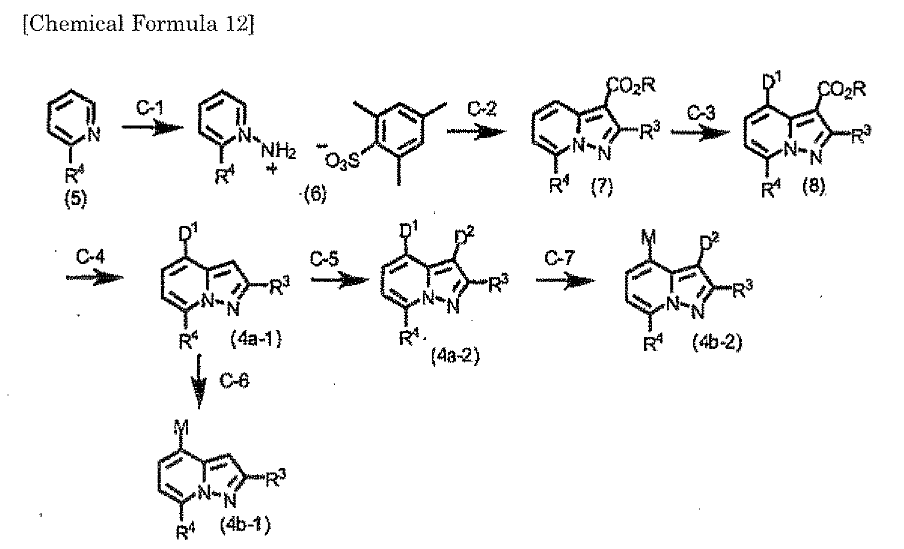

In the synthetic route scheme C, the compound represented by the following general formula (6):

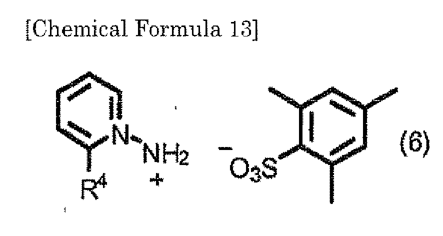

[wherein R⁴ is the same as that defined above]
can be prepared by acting O-mesitylenesulfonylhydroxylamine (hereunder referred to as "MSH") on a compound represented by the following general formula (5) (Step C-1):

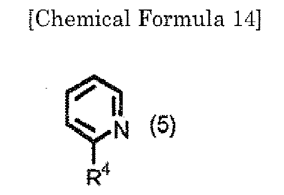

[wherein R⁴ is the same as that defined above].

This reaction can preferably be carried out by dissolving the compound of Formula (5) in methylene chloride and then acting a solution of MSH in methylene chloride on the compound of Formula (5) at a temperature ranging from 0°C to ordinary temperature.

In the synthetic route scheme C, the compound represented by the following general formula (7):

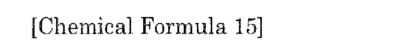

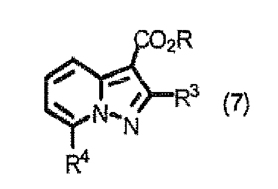

[wherein R represents an alkyl group having 1 to 6 carbon atoms or a benzyl group; R³ and R⁴ are the same as those defined above], can be prepared by acting, on a compound represented by the general formula (6), a compound represented by the following general formula (9), in the presence of a base:

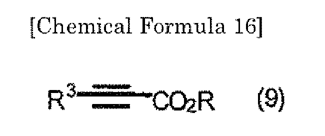

[wherein R and R³ are the same as those defined above], (Step C-2).

This reaction can be carried out at a temperature ranging from 0°C to ordinary temperature and preferably ordinary temperature in the presence of an inorganic base such as sodium hydrogen carbonate, sodium carbonate, potassium hydrogen carbonate or potassium carbonate, or an organic base such as triethylamine and preferably potassium carbonate, while using, as a reaction solvent such as methanol, ethanol, 1,4-dioxane, dimethylsulfoxide (DMSO), DMF, THF, toluene, benzene, cyclohexane, cyclopentane, methylene chloride, chloroform or acetonitrile and preferably DMF.

In the synthetic route scheme C, the compound represented by the following general formula (8):

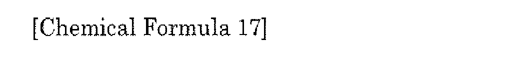

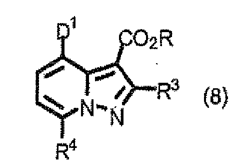

[wherein D¹ represents a halogen atom, and R, R³ and R⁴ are the same as those defined above]
can be prepared by the halogenation of a compound of Formula (7) (Step C-3).

This reaction can be carried out at a temperature ranging from 0°C to 80°C, while using N-chlorosuccinimide (NCS), N-bromosuccinimide (NBS), N-iodosuccinimide (NIS), bromine or iodine and also using, as a reaction solvent, acetic acid, DMF, methylene chloride, chloroform or acetonitrile and preferably acetonitrile.

In the synthetic route scheme C, the compound represented by the following general formula (4a-1):

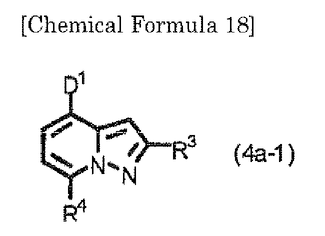

[wherein R³, R⁴ and D¹ are the same as those defined above]
can be prepared by hydrolyzing a compound represented by Formula (8) and then subjecting the resultant hydrolyzate to a decarboxylation reaction (Step C-4).

This reaction can be carried out by heating, with refluxing, in hydrobromic acid or hydrogen bromide-containing acetic acid. In addition, it is also possible to carry out the reaction by acting, on the compound of Formula (8), an aqueous solution of sodium hydroxide, an aqueous solution of potassium hydroxide or an aqueous solution of lithium hydroxide at a temperature ranging from ordinary temperature to the refluxing by heat, in a reaction solvent such as methanol, ethanol, THE, DMSO, DMF or 1,4-dioxane to thus hydrolyze the compound into a carboxylic acid and then further continuing the reaction at a temperature ranging from 140 to 160°C in benzene, chlorobenzene, dichlorobenzene, bromobenzene, toluene or xylene; or by reacting the compound of Formula (8) in ethanol or 1,4-dioxane while adding a 2 to 10% sulfuric acid aqueous solution at 100°C. Alternatively, the reaction can be carried out in a 50% sulfuric acid solution while heating at 100°C and stirring.

In the synthetic route scheme C, the compound represented by the following general formula (4a-2):

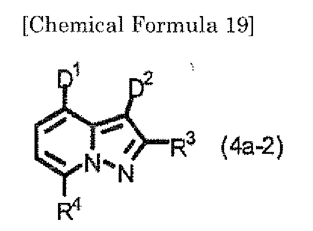

[wherein D² represents a halogen atom, R³, R⁴ and D¹ are the same as those defined above]
can be prepared by the halogenation of a compound of Formula (4a-1) (Step C-5).

This reaction can be carried out by the same method used in the Step C-3.

In the synthetic route scheme C, the compound represented by the following general formula (4b-1):

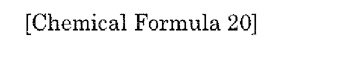

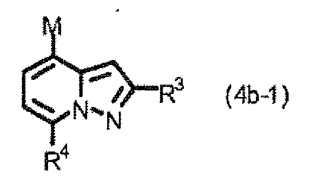

[wherein R³, R⁴ and M are the same as those defined above]
can be prepared by stannylation or boration of a compound represented by the general formula (4a-1) (Step C-6).

When M represents -SnBus or -SnMe₃, the reaction can be carried out by acting, on the compound of Formula (4a-1), an organometal reagent such as n-butyl lithium, s-butyl lithium, t-butyl lithium or isopropyl magnesium chloride in a solvent such as toluene, THF, diethyl ether or dioxane and then acting, on the resultant product, a tin-containing reagent such as tributyl tin chloride or trimethyl tin chloride at a temperature ranging from -78°C to ordinary temperature. Alternatively, it is also possible to prepare the compound by acting, on the compound of Formula (4a-1), bis(tributyltin) or bis(trimethyltin) at a temperature ranging from ordinary temperature to 140°C, in the presence of a palladium catalyst such as Pd(PPh₃)₄ or PdCl₂(dppf), in a solvent such as toluene, THF, dioxane, DMF or DMSO.

In case where M represents -B(OH)₂, the compound can be prepared by acting, on the compound of Formula (4a-1), an organometal reagent such as n-butyl lithium, s-butyl lithium, t-butyl lithium or isopropyl magnesium chloride, preferably n-butyl lithium, in a solvent such as toluene, THF, diethyl ether or dioxane, subjecting the resultant product to a reaction with a borane reagent such as trimethoxy borane or triisopropoxy borane at a temperature ranging from -78°C to ordinary temperature and then subjecting the product to acid hydrolysis using, for instance, hydrochloric acid. When M is -B(OMe)₂, the desired compound can be prepared by acting, on the compound of Formula (4a-1), an organometal reagent such as n-butyl lithium, s-butyl lithium, t-butyl lithium or isopropyl magnesium chloride, preferably n-butyl lithium, in a solvent such as toluene, THF, diethyl ether or dioxane, and then subjecting the resultant product to a reaction with trimethoxy borane at a temperature ranging from -78°C to ordinary temperature. When M represents a group represented by the following formula:

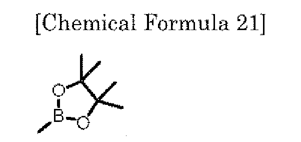

the desired compound can be prepared by acting bis(pinacolato)dibarou or pinacol borane at a temperature ranging from ordinary temperature to 140°C, in a solvent such as toluene, THF, dioxane, DMF or DMSO and preferably DMSO, in the presence of a palladium catalyst such as Pd(PPh₃)₄ or PdCl₂(dppf), while using a base such as potassium acetate, sodium acetate or potassium 2-ethylhexanoate. In addition, when M represents a group represented by the following formula:

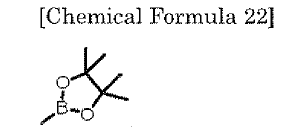

the desired compound can be prepared by acting pinacol on a compound in which M represents -B(OH)₂ or -B(OMe)₂. Moreover, when M represents -B(OH)₂, the desired compound can be prepared by subjecting a compound in which M represents a group represented by the following formula:

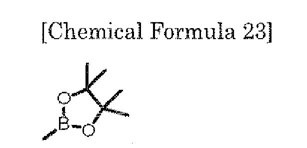

to a hydrolysis reaction using a sodium hydroxide aqueous solution or hydrochloric acid.
In the synthetic route scheme C, the compound represented by the following general formula (4b-2):

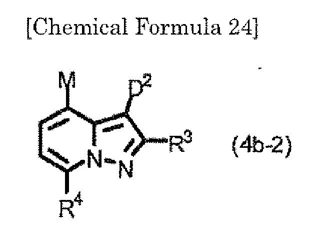

[wherein R³, R⁴, D² and M are the same as those defined above]
can be prepared by stannylation or boration of the compound of Formula (4a-2) (Step C-7).

The reaction can be carried out by the same method used in the step C-6.

### [Synthetic Route Scheme C']

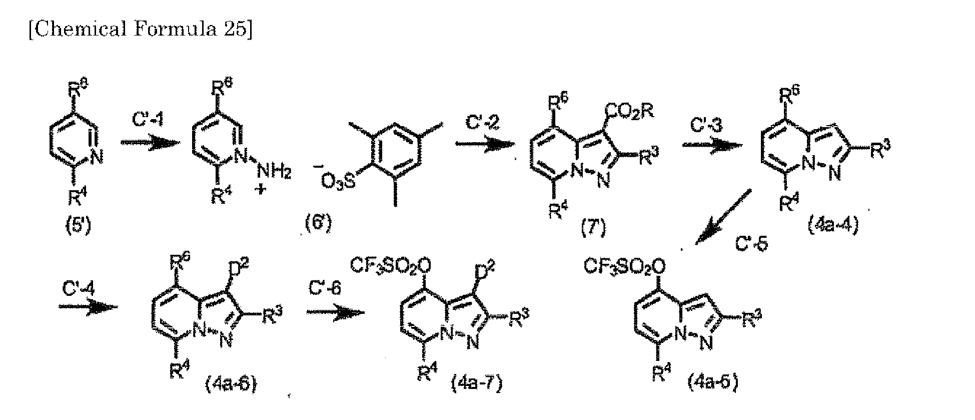

In the synthetic route scheme C', the compound represented by the following general formula (6):

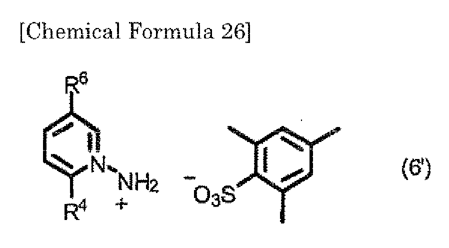

[wherein R⁶ represents a halogen atom or a hydroxyl group carrying a protecting group (P¹) (wherein P¹ represents a protecting group such as methoxymethyl group, methoxyethoxymethyl group, tetrahydropyranyl group, benzyl group, p-methoxy benzyl group, t-butyldimethylsilyl group, t-butyldiphenylsilyl group or triiso propylsilyl group), and R⁴ is the same as that defined above], can be prepared by acting MSH on a compound represented by the following general formula (5'):

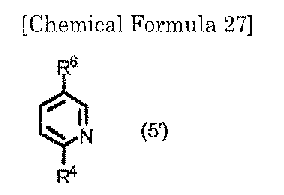

[wherein R⁴ and R⁶ are the same as those defined above] (Step C'-1).

The reaction can be carried out by the same method used in the step C-1.
In the synthetic route scheme C', the compound represented by the following general formula (7'):

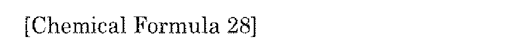

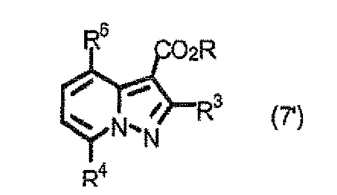

[wherein R³, R⁴, R⁶ and R are the same as those defined above]
can be prepared by acting a compound of Formula (6') on a compound of Formula (9) in the presence of a base (Step C'-2).

The reaction can be carried out by the same method used in the step C-2.
In the synthetic route scheme C', the compound represented by the following general formula (4a-4):

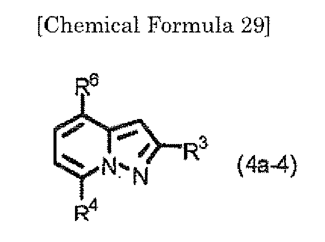

[wherein R³, R⁴ and R⁶ are the same as those defined above]
can be prepared by hydrolyzing a compound represented by Formula (7') and then subjecting the resultant hydrolyzate to a decarboxylation reaction (Step C'-3).

When R⁶ represents a halogen atom, the reaction can be carried out by the same method used in the step C-4; when R⁶ represents a hydroxyl group carrying a protecting group, the reaction can be carried out by acting a sodium hydroxide aqueous solution, a potassium hydroxide aqueous solution or a lithium hydroxide aqueous solution on the compound, in a solvent such as methanol, ethanol, THF, DMSO, DMF or 1,4-dioxane at a temperature ranging from ordinary temperature to reflux temperature to thus hydrolyze the compound into the corresponding carboxylic acid and then subjecting the system to a reaction at a temperature ranging from 80 to 160°C in a solvent such as benzene, chlorobenzene, dichlorobenzene, bromobenzene, toluene or xylene.
In the synthetic route scheme C', the compound represented by the following general formula (4a-6):

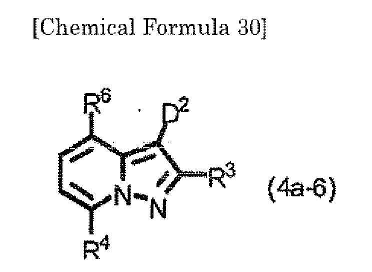

[wherein R³, R⁴, R⁶ and D² are the same as those defined above]
can be prepared by halogenating a compound represented by Formula (4a-4) (Step C'-4).

The reaction can be carried out by the same method used in the step C-3.
In the synthetic route scheme C', the compound represented by the following general formula (4a-5):

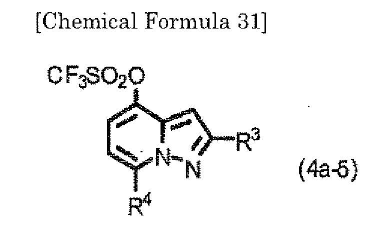

[wherein R³ and R⁴ are the same as those defined above]
can be prepared by removing the protecting group of the hydroxyl group attached to a compound represented by Formula (4a-4) in which R⁶ represents a hydroxyl group carrying a protecting group and then converting the resultant hydroxyl group into a trifluoro methanesulfonyloxy group (Step C'-5).
The deprotecting reaction can be carried out using hydrogen chloride-containing methanol, ethanol, ethyl acetate or diethyl ether at a temperature ranging from 0°C to ordinary temperature when the protecting group is a methoxymethyl group or a tetrahydropyranyl group. The deprotecting reaction can be carried out according to the catalytic reduction technique when the protecting group is a benzyl group or a p-methoxybenzyl group. In addition, the deprotecting reaction can be carried out by reacting a protected compound with, for instance, 2,3-dichloro-5,6-dicyano-p-benzoquinone (DDQ) or ceric ammonium nitrate (CAN) at a temperature ranging from 0°C to ordinary temperature in a solvent such as dichloromethane or acetonitrile, when the protecting group is a p-methoxybenzyl group. When the protecting group is a silyl protecting group such as a t-butyldimethylsilyl group, a t-butyldiphenylsilyl group or a triisopropylsilyl group, the deprotecting reaction can be carried out in a solvent such as acetonitrile or THF at a temperature ranging from 0°C to ordinary temperature, while using potassium fluoride, cesium fluoride or tetrabutylammonium fluoride.

Trifluoromethanesulfonylation of the hydroxyl group can be carried out by acting, on the hydroxyl group, trifluoromethanesulfonyl chloride or trifluoromethanesulfonic acid anhydride at a temperature ranging from -78 °C to ordinary temperature, in the presence of a base such as triethylamine or diisopropylethylamine in a solvent such as dichloromethane.
In the synthetic route scheme C', the compound represented by the following general formula (4a-7):

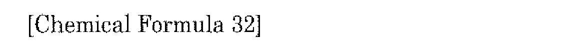

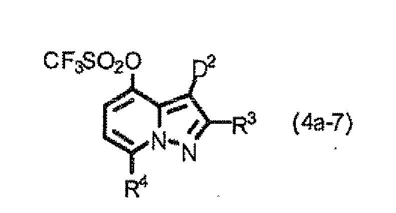

[wherein R³, R⁴ and D² are the same as those defined above]
can be prepared by removing the protecting group on the hydroxyl group of a compound of Formula (4a-6) and then converting the resultant hydroxyl group into trifluoromethanesulfonyl group (Step C'-6).

The reaction can be carried out by the same method used in the step C'-5.
In the synthetic route scheme C, the compounds among those represented by the formula (4a-1) or (4a-2), in which R⁴ represents an amino group or an alkyl amino group having 1 to 6 carbon atoms, in other words, each of the compounds represented by the following general formula (4a-1-1.) or (4a-2-1):

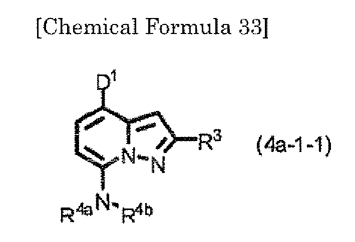

[wherein R^{4a} and R^{4b} represent a hydrogen atom or an alkyl group having 1 to 6 carbon atoms, and R³ and D¹ are the same as those defined above], or

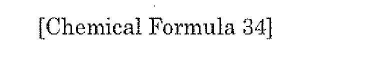

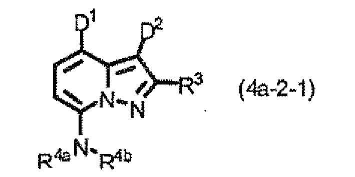

[wherein R³, R^{4a}, R^{4b}, D¹ and D² are the same as those defined above]
can be prepared according to the following synthetic route scheme C":

### [Synthetic Route Scheme C"]

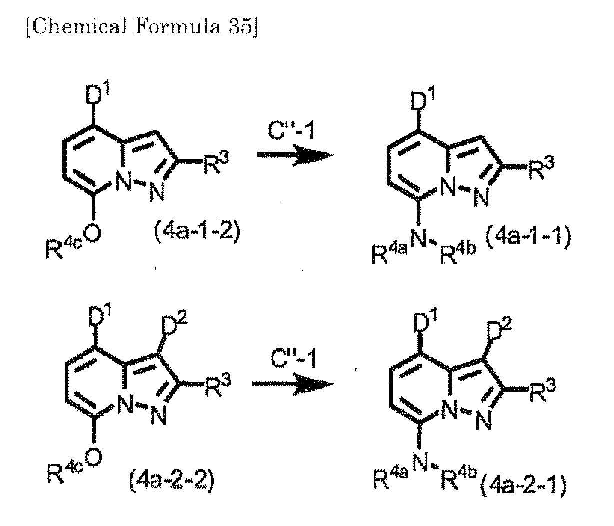

Regarding the preparation method, those compounds can be prepared by acting, on the compounds among those represented by the formula (4a-1) or (4a-2), appearing in the synthetic route scheme C, in which R⁴ represents an alkoxy group having 1 to 6 carbon atoms, in other words, each of the compounds represented by the following general formulas (4a-1-2) or (4a-2-2):

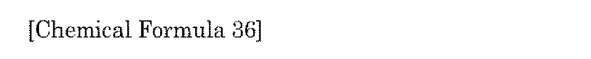

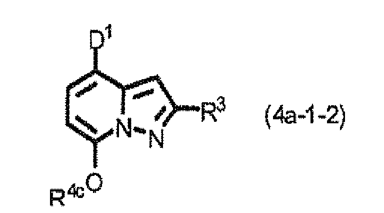

[wherein R^{4c} represents an alkyl group having 1 to 6 carbon atoms and R³ and D¹ are the same as those defined above]; or
[or]

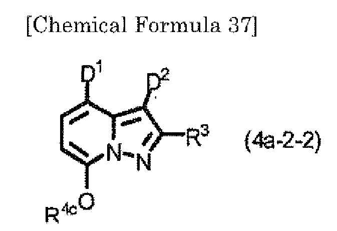

[wherein R³, R^{4c}, D¹ and D² are the same as those defined above]
a compound represented by the following general formula (10):

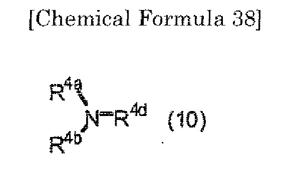

[wherein R^{4d} represents a hydrogen atom or a formyl group, and R^{4a} and R^{4b} are the same as those defined above] (Step C"-1).

This reaction can be carried out at a temperature ranging from ordinary temperature to 100°C, in a solvent such as THF, DMF or DMSO, in the presence or absence of a base such as lithium hydride, sodium hydride, potassium hydride, n-butyl lithium, s-butyl lithium or t-butyl lithium.
In the synthetic route schemes A and B, the compounds having a triazolopyridine residue as the Heterocycle moiety, among those represented by the general formula (4a) and (4b) can be prepared according to the following synthetic route scheme D:

### [Synthetic Route Scheme D]

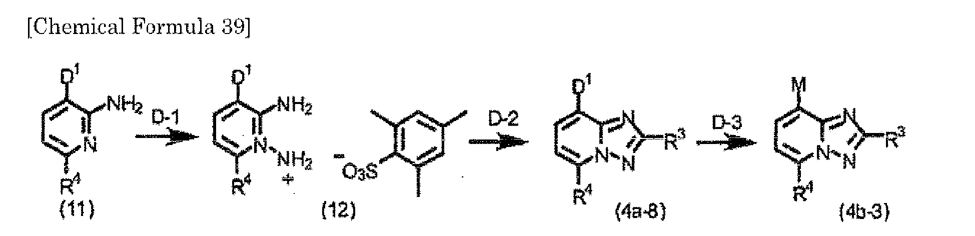

In the synthetic route scheme D, the compound represented by the following general formula (12):

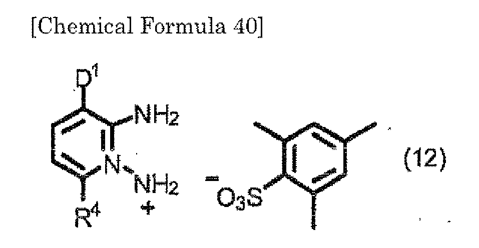

[wherein R⁴ and D¹ are the same as those defined above]
can be prepared by acting MSH on a compound represented by the following general formula (11):

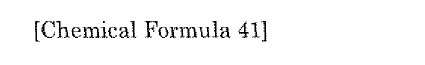

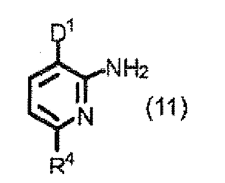

[wherein R⁴ and D¹ are the same as those defined above], (Step D-1).

This reaction can be carried out by the same method used in the step C-1.
In the synthetic route scheme D, the compound represented by the following general formula (4a-8):

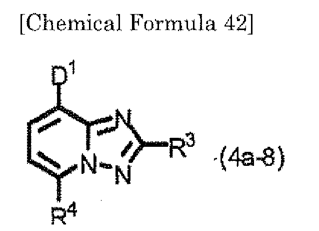

[wherein R³, R⁴ and D¹ are the same as those defined above]
can be prepared by acting, on the compound represented by the general formula (12), a compound represented by the following general formula (13):

[Chemical Formula 43] (R³CO)₂O (13)

[wherein R³ is the same as that defined above]
in the presence of a base (Step D-2).

This reaction can be carried out at a temperature ranging from ordinary temperature to the heat refluxing temperature in the presence of a base such as triethylamine, sodium hydroxide, potassium hydroxide or potassium carbonate, preferably triethylamine, while using a solvent such as benzene, toluene, xylene, methanol or ethanol.
In the synthetic route scheme D, the compound represented by the following general formula (4b-3):

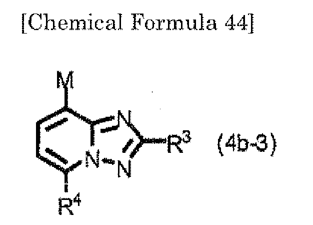

[wherein R³, R⁴ and M are the same as those defined above]
can be prepared by stannylation or boration of a compound represented by the general formula (4a-8) (Step D-3).

This reaction can be carried out by the same method used in the step C-6.
In the synthetic route schemes A and B, the compounds having a imidazopyridine residue as the Heterocycle moiety, among those represented by the general formula (4a) and (4b) can be prepared according to the following synthetic route scheme E:

### [Synthetic Route Scheme E]

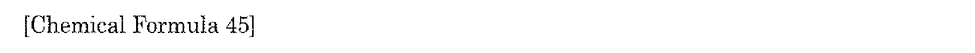

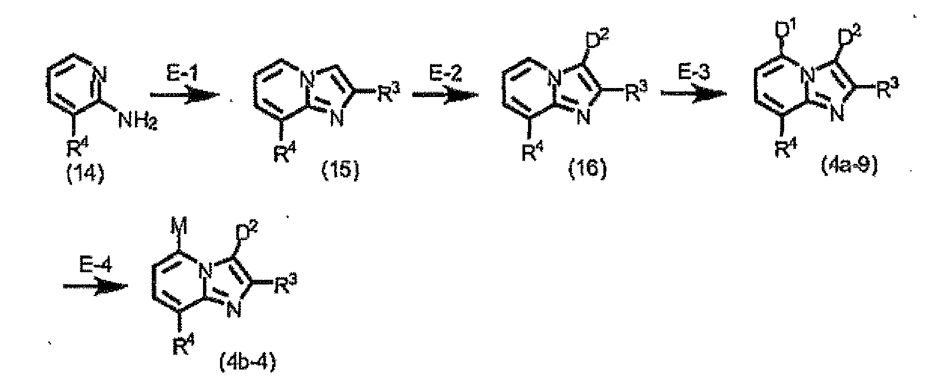

In the synthetic route scheme E, the compound represented by the following general formula (15):

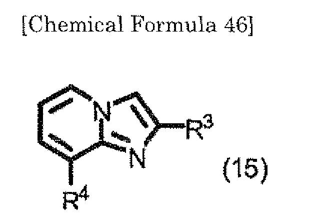

[wherein R³ and R⁴ are the same as those defined above]
can be prepared by acting, on a compound represented by the following general formula (14):

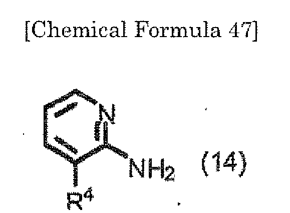

[wherein R⁴ is the same as that defined above]
a compound represented by the following general formula (17):

[Chemical Formula 48] R₃COCH₂X¹ (17)

[wherein X¹ represents a chlorine, bromine or iodine atom and R³ is the same as that defined above]
in the presence or absence of a base (Step E-1).

This reaction can be performed at a temperature ranging from ordinary temperature to heat refluxing temperature in a solvent such as benzene, toluene, xylene, methanol or ethanol, and, when a base is used, using a base such as triethylamine, sodium hydrogen carbonate, sodium carbonate, sodium hydroxide, potassium hydrogen carbonate, potassium carbonate or potassium hydroxide.
In the synthetic route scheme E, the compound represented by the following general formula (16):

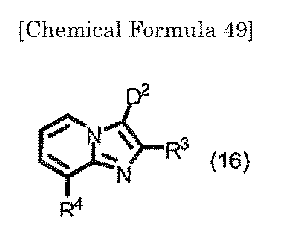

[wherein R³, R⁴ and D² are the same as those defined above]
can be prepared by the halogenation of a compound represented by the general formula (15) (Step E-2).

The reaction can be carried out by the same method used in the step C-3.
In the synthetic route scheme E, the compound represented by the following general formula (4a-9):

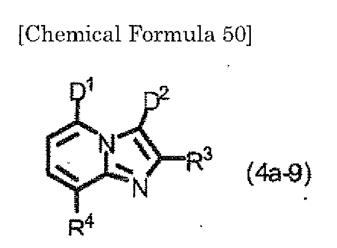

[wherein R³, R⁴, D¹ and D² are the same as those defined above]
can be prepared by the halogenation of a compound represented by the general formula (16) (Step E-3).

The reaction can be carried out by the same method used in the step C-3.
In the synthetic route scheme E, the compound represented by the following general formula (4b-4):

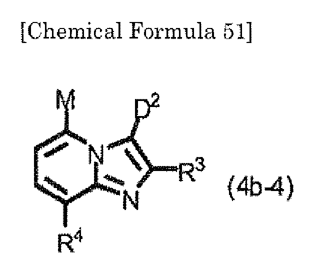

[wherein R³, R⁴, D² and M are the same as those defined above]
can be prepared by stannylation or boration of the compound of Formula (4a-9) (Step E-4).

The reaction can be carried out by the same method used in the step C-6.
In the synthetic route schemes A and B, the compounds having a benzothiazole residue as the Heterocycle moiety, among those represented by the general formula (4a) and (4b) can be prepared according to the following synthetic route scheme F:

### [Synthetic Route Scheme F]

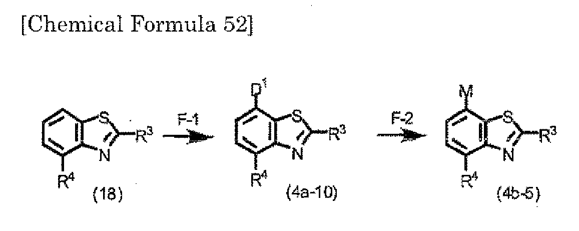

In the synthetic route scheme F, the compound represented by the following general formula (4a-10):

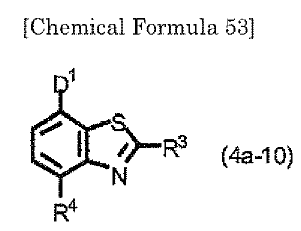

[wherein R³, R⁴ and D¹ are the same as those defined above]
can be prepared by the halogenation of a compound represented by the following general formula (18):

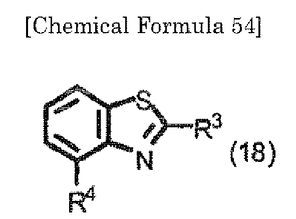

[wherein R³ and R⁴ are the same as those defined above], (Step F-1).

The reaction can be carried out by the same method used in the step C-3.
In the synthetic route scheme F, the compound represented by the following general formula (4b-5):

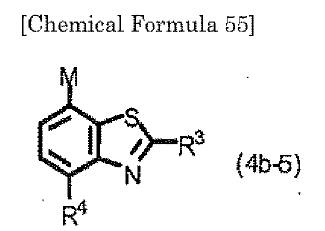

[wherein R³, R⁴ and M are the same as those defined above]
can be prepared by stannylation or boration of the compound of Formula (4a-10) (Step F-2).

The reaction can be carried out by the same method used in the step C-6.
In the synthetic route schemes A and B, the compounds having a benzofuran or benzothiophene residue as the Heterocycle moiety, among those represented by the general formula (4a) and (4b) can be prepared according to the following synthetic route scheme G:

### [Synthetic Route Scheme G]

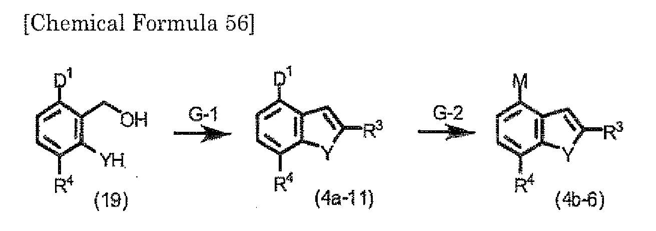

In the synthetic route scheme G, the compound represented by the following general formula (4a-11):

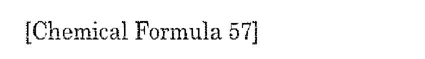

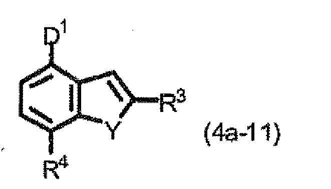

[wherein R³, R⁴, D¹ and Y are the same as those defined above]
can be prepared by subjecting a compound represented by the following general formula (19) to a reaction with triphenylphosphonium bromide:

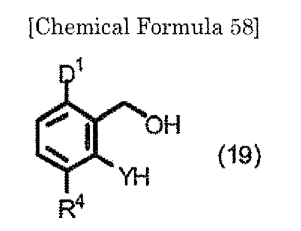

[wherein R⁴, D¹ and Y are the same as those defined above]
and then subjecting the resultant product to a reaction with a compound of Formula (13) (Step G-1).

In this reaction, it is preferred that the compound (19) is reacted with triphenylphosphonium bromide while refluxing by heating, using a solvent such as acetonitrile, THF, 1,4-dioxane or ethyl acetate, preferably acetonitrile, followed by the replacement of the reaction solvent with toluene, benzene or xylene, preferably toluene, the addition of triethylamine and a compound of Formula (13), and then reacting them while refluxing by heating.
In the synthetic route scheme G, the compound represented by the following general formula (4b-6):

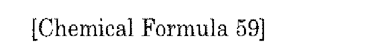

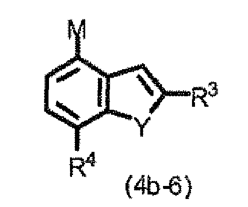

[wherein R³, R⁴, M and Y are the same as those defined above]
can be prepared by stannylation or boration of the compound of Formula (4a-11) (Step G-2).

The reaction can be carried out by the same method used in the step C-6.
In the synthetic route schemes A and B, the compounds having a benzoxazolone residue as the Heterocycle moiety, among those represented by the general formula (4a) and (4b) can be prepared according to the following synthetic route scheme H:

### [Synthetic Route Scheme H]

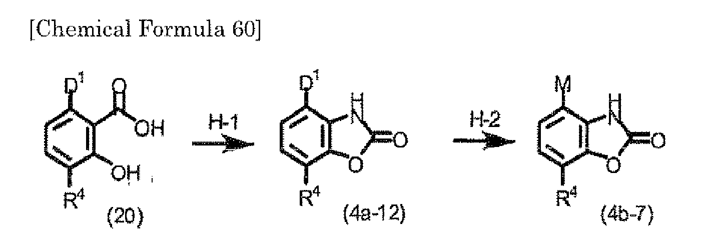

In the synthetic route scheme H, the compound represented by the following general formula (4a-12):

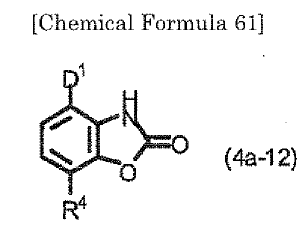

[wherein R⁴ and D¹ are the same as those defined above]
can be prepared by the use of a compound represented by the following general formula (20), (Step H-1):

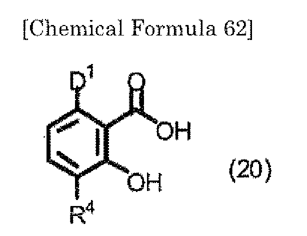

[wherein R⁴ and D¹ are the same as those defined above].

This reaction can be carried out by adding diphenylphosphoryl azide to the compound of Formula (20), in a solvent such as toluene or xylene and then heating them at a temperature ranging from 50°C to 150°C in the presence of a base such as triethylamine or diisopropylethylamine.
In the synthetic route scheme H, the compound represented by the following general formula (4b-7):

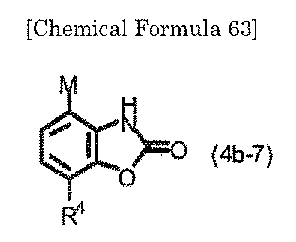

[wherein R⁴ and M are the same as those defined above]
can be prepared by stannylation or boration of the compound of Formula (4a-12) (Step H-2).

The reaction can be carried out by the same method used in the step C-6.
In the synthetic route schemes A and B, the compounds having a quinazoline residue as the Heterocycle moiety, among those represented by the general formula (4a) and (4b) can be prepared according to the following synthetic route scheme J:

### [Synthetic Route Scheme J]

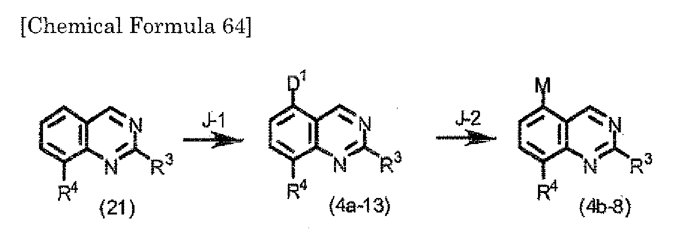

In the synthetic route scheme J, the compound represented by the following general formula (4a-13):

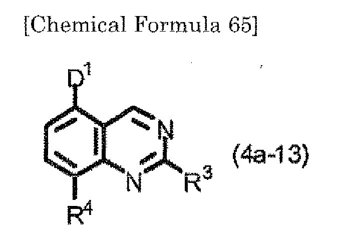

[wherein R³, R⁴ and D¹ are the same as those defined above]
can be prepared by halogenating the compound represented by the following general formula (21):

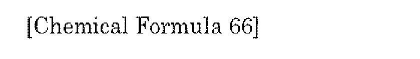

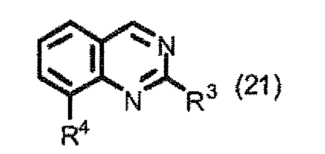

[wherein R³ and R⁴ are the same as those defined above], (Step J-1).

The reaction can be carried out by the same method used in the step C-3.
In the synthetic route scheme J, the compound represented by the following general formula (4b-8):

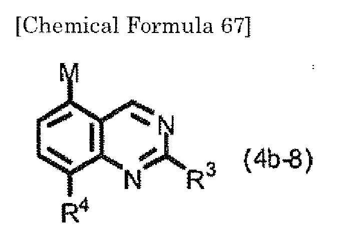

[wherein R³, R⁴ and M are the same as those defined above]
can be prepared by stannylation or boration of the compound of Formula (4a-13) (Step J-2).

The reaction can be carried out by the same method used in the step C-6.
In the synthetic route schemes A and B, the compounds having a 2H-benzo[e][1,3]oxazin-4(3H)-one residue as the Heterocycle moiety, among those represented by the general formula (4a) and (4b) can be prepared according to the following synthetic route scheme K:

### [Synthetic Route Scheme K]

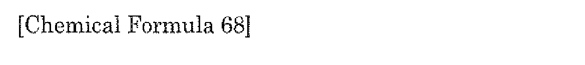

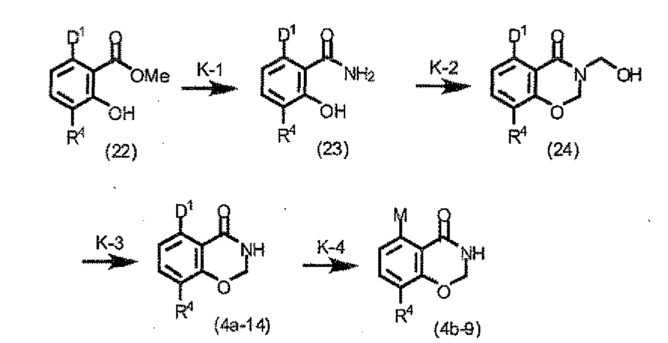

In the synthetic route scheme K, the compound represented by the following general formula (23):

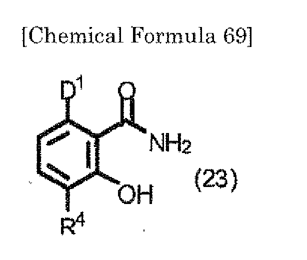

[wherein R⁴ and D¹ are the same as those defined above]
can be prepared by amidation of the compound represented by the following general formula (22):

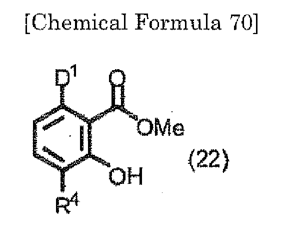

[wherein R⁴ and D¹ are the same as those defined above] (Step K-1).
This reaction can be carried out by acting aqueous ammonia on the compound of Formula (22) at a temperature ranging from ordinary temperature to 100°C.
In the synthetic route scheme K, the compound represented by the following general formula (24):

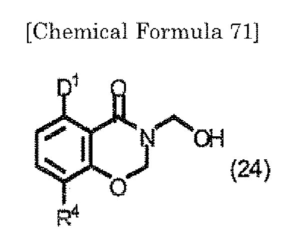

[wherein R⁴ and D¹ are the same as those defined above]
can be prepared through the reaction of a compound of Formula (23) with formaldehyde (Step K-2).

This reaction is preferably carried out by acting an aqueous formalin solution on the compound in formic acid under the heat refluxing condition.
In the synthetic route scheme K, the compound represented by the following general formula (4a-14):

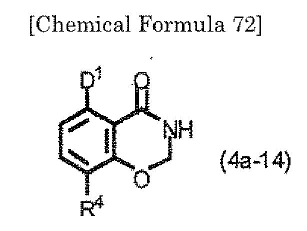

[wherein R⁴ and D¹ are the same as those defined above]
can be prepared by subjecting a compound of Formula (24) to the treatment for the removal of its hydroxymethyl group (Step K-3).

This reaction can preferably be carried out in a solvent such as toluene or xylene, while heating the reaction system to a temperature ranging from 60°C to 150°C.
In the synthetic route scheme K, the compound represented by the following general formula (4b-9):

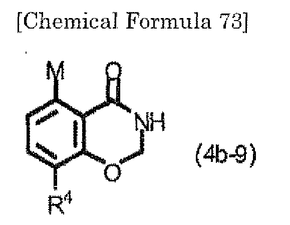

[wherein R⁴ and M are the same as those defined above]
can be prepared by stannylation or boration of the compound of Formula (4a-14) (Step K-4).

The reaction can be carried out by the same method used in the step C-6.
In the synthetic route schemes A and B, the compounds having a 2H-benzo[b][1,4]oxazin-3(4H)-one residue as the Heterocycle moiety, among those represented by the general formula (4a) and (4b) can be prepared according to the following synthetic route scheme L:

### [Synthetic Route Scheme L]

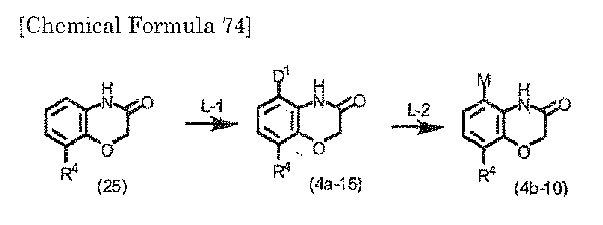

In the synthetic route scheme L, the compound represented by the following general formula (4a-15):

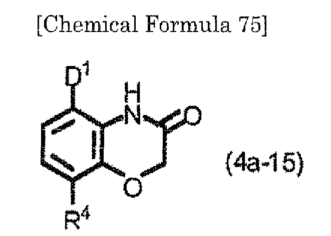

[wherein R⁴ and D¹ are the same as those defined above]
can be prepared by the halogenation of a compound represented by the following general formula (25):

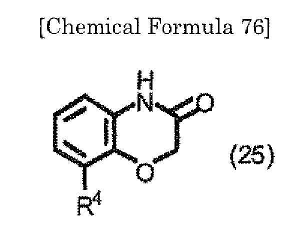

[wherein R⁴ is the same as that defined above] (Step L-1).

The reaction can be carried out by the same method used in the step C-3.
In the synthetic route scheme L, the compound represented by the following general formula (4b-10):

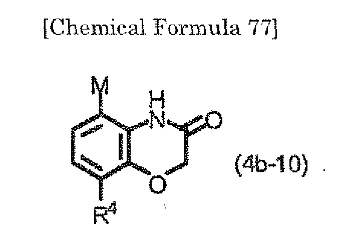

[wherein R⁴ and M are the same as those defined above]
can be prepared by stannylation or boration of the compound of Formula (4a-15) (Step L-2).

The reaction can be carried out by the same method used in the step C-6.
Moreover, among the compounds represented by the general formula (1), those represented by the following general formula (1c) or those represented by the general formula (1), in which n is 1:

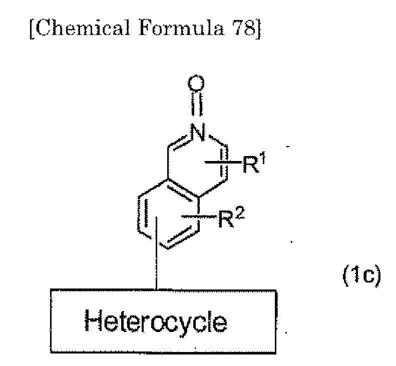

[wherein R¹, R² and Heterocycle are the same as those defined above]
can be prepared by the oxidation of a compound represented by the general formula (1), in which n is 0 or the compound represented by the following general formula (1b):

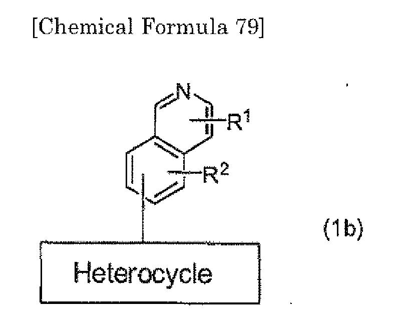

[wherein R¹, R² and Heterocycle are the same as those defined above].

This reaction can be carried out by the oxidation of the compound of Formula (1b) with an oxidizing agent such as an aqueous hydrogen peroxide solution, tert-butyl hydroperoxide, peracetic acid, permaleic acid, pertrifluoroacetic acid, m-chloroperbenzoic acid, magnesium monoperoxyphthalate or sodium perborate at a temperature ranging from 0°C to 150°C in a solvent such as water, acetic acid, methylene chloride, chloroform or 1,2-dichloroethane.
When using the compound or optically active derivative or pharmaceutically acceptable salt thereof according to the present invention as pharmaceutical agents, it can be used, as need arises, in any optimum form selected from, for instance, a solid composition, a liquid composition or other compositions. The pharmaceutical agent according to the present invention can be prepared by incorporating, into the compound of the present invention, a pharmaceutically acceptable carrier. Specifically, additives such as currently used excipients, bulking agents, binders, disintegrating agents, coating agents, sugar-coating agents, pH-adjusting agents, solubilizing agents, or aqueous or non-aqueous solvents are added to the compound of the present invention and then the resultant mixture can be formed into any dosage form such as tablets, pills, capsules, granules, powders, triturates, liquids, emulsions, suspensions and injections according to the commonly used drug-manufacturing techniques.

The dose of the compound or optically active derivative or pharmaceutically acceptable salt thereof according to the present invention may vary depending on various factors such as the kinds of diseases and symptoms, body weight, age and sexes of patients to be treated and routes of administration, but the dose for the adult preferably ranges from about 0.01 to about 1000 mg/kg (body weight)/day and more preferably about 0.5 to about 200 mg/kg (body weight)/day for the oral administration and it can be administered once a day or several times a day.

### Example

The present invention will now be described with reference to the following specific examples, but the scope of the present invention is by no means limited to these specific examples.

Incidentally, the intermediates used for the synthesis and cited in the following Examples were those disclosed in the following patent documents: The pamphlets of Domestically re-published WO 98/14448; JP-A-10-109988; JP-A-2006-117647; JP-A-2006-169138; WO 2006/095666; JP-A-2007-091597; JP-A-2008-024599; JP-A-2008-063265; JP-A-2008-069144; WO 2008/026687; WO 2008/029829; and WO 2008/029882.

### Example 1: N-Amino-2-amino-3-bromo-6-methoxypyridinium mesitylenesulfonate

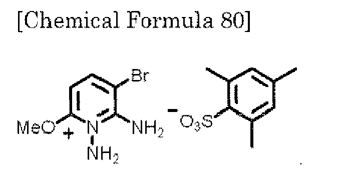

Mesitylsulfonylacetohydroxamic acid ethyl ester (25.3 g) was dissolved in 1,4-dioxane (35 mL), a 70% perchloric acid solution (13 mL) was added to the resultant solution at 0°C and then the mixture was stirred for 30 minutes. After the addition of cold water to the reaction mixture, the solids thus precipitated were collected by filtration and then dissolved in methylene chloride. After the removal of the aqueous layer through the liquid-separatory operation, the remaining methylene chloride layer was washed with saturated brine, followed by the drying of the layer over anhydrous sodium sulfate. The filtrate obtained after the filtration of the methylene chloride layer was added to a solution of 2-amino-3-bromo-6-methoxypyridine (15.0 g) in methylene chloride (100 mL) at 0°C and the resultant mixture was stirred at ordinary temperature for one hour. The solvent was distilled off under reduced pressure, diethyl ether was added to the resultant residue and the crystals thus precipitated were collected by filtration to give a desired compound (27.3g) as yellow powder.
¹H NMR (DMSO-d₆, 400 MHz): δ 2.14 (3H, s), 2.47 (6H, s), 4.06 (3H, s), 5.73 (1H, s), 6.28 (2H, s), 6.45 (1H, d, J = 8.6 Hz), 6.71 (2H, s), 8.20 (1H, d, J = 8.6 Hz), 8.40 (2H, s).

### Example 2: N-Amino-2-methoxypyridinium mesitylenesulfonate

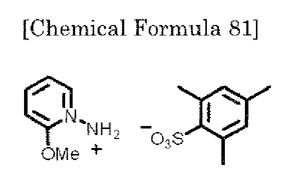

The same procedures as used in Example 1 were carried out except for using 2-methoxypyridine (81.4 g) to give a crude desired compound (66.9 g). The compound was used in the subsequent process without any further purification.

### Example 3: Ethyl 7-methoxy-2-trifluoromethylpyrazolo[1,5-a]pyridine-3- carboxylate

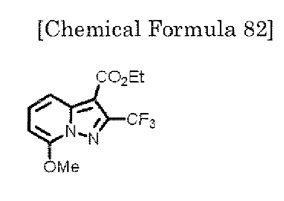

The compound prepared in Example 2 (60.1 g) and 4,4,4-trifluoro-2-butynoic acid ethyl ester (30.8 g) were dissolved in DMF, (500 mL), then potassium carbonate (51.1 g) was added to the resultant solution, and the resultant mixture was stirred at ordinary temperature for 14 hours. After the solvent was distilled off under reduced pressure, the resultant residue was dissolved in ethyl acetate, the solution was filtered through Celite to remove the insoluble material, water was added to the resultant filtrate and the mixture was extracted three times with ethyl acetate. The extracted layer was washed with saturated brine, dried over anhydrous sodium sulfate and then the solvent was distilled off under reduced pressure. The resultant residue was purified by the silica gel column chromatography (hexane: ethyl acetate = 10:1→1.5:1) to give a desired compound (15.2 g) as yellow powder.
¹H NMR (CDCl₃, 400 MHz): δ 1.42 (3H, t, J = 7.3 Hz), 4.20 (3H, s), 4.42 (2H, q, J= 7.3 Hz), 6.40 (1H, d,J = 7.6 Hz), 7.51 (1H, dd, J = 8.6, 7.6 Hz), 7.91 (1H, d, J= 8.6 Hz).

### Example 4: Ethyl 4-bromo-7-methoxy-2-trifluoromethylpyrazolo[1,5-a]pyridhae-3-carboxylate:

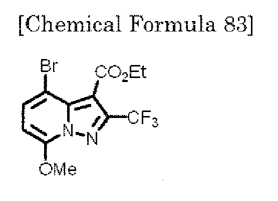

The compound (13.9 g) disclosed in Example 12 of WO 2006/095666 was dissolved in acetonitrile (400 mL), NBS (12.0 g) was added to the resultant solution and the resultant mixture was stirred at 70°C for 5 hours. A 10% sodium thiosulfate aqueous solution was added to the mixture, the acetonitrile was distilled off under reduced pressure, the residue thus obtained was extracted three times with ethyl acetate, the extracted layer was washed with saturated brine, then it was dried over anhydrous sodium sulfate and the solvent was distilled off under reduced pressure. The resultant residue was purified by the silica gel column chromatography (hexane: ethyl acetate = 3:1→1:1) to give a desired compound (10.6 g) as yellow powder.
¹H NMR (CDCl₃ , 400 MHz): δ 1.42 (3H, t, J = 7.3 Hz), 4.19 (3H, s), 4.44 (2H, q, J = 7.3 Hz), 6.25 (1H, d, J = 7.9 Hz), 7.59 (1H, d, J = 7.9 Hz).

### Example 5: 4-Bromo-7-methoxy-2-trifluoromethylpyrazolo[1,5-a]pyridine:

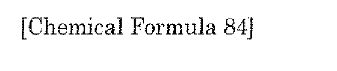

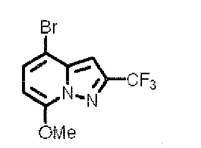

The compound (10.2 g) prepared in Example 4 was dissolved in a methanol (140 mL)-water (70 mL) mixed solvent, potassium hydroxide (4.68 g) was added to the solution and the mixture was stirred for 3.5 hours under heated and refluxed conditions. The pH value of the mixture was adjusted to about 2 by the addition of a 1.0 mol/L hydrochloric acid solution, the mixture was then extracted three times with ethyl acetate, the extract was washed with saturated brine, dried over anhydrous sodium sulfate and then the solvent was distilled off under reduced pressure to give a crude carboxylic acid (9.34 g). The resultant carboxylic acid (9.34 g) was dissolved in ethanol (140 mL), concentrated sulfuric acid (7 mL) was added to the solution and then the mixture was stirred for 4 hours under heat refluxing condition. A 10% sodium hydroxide aqueous solution was added to the mixture to adjust the pH value thereof to 7 and the ethanol was distilled off under reduced pressure. Further a 10% sodium hydroxide aqueous solution was added to the mixture to adjust the pH value thereof to 8 to 9, the mixture was extracted three times with ethyl acetate, the extracted layer was washed with saturated brine and then dried over anhydrous sodium sulfate. The filtrate obtained after the filtration of the extracted layer was passed through aminated silica gel, the solvent was distilled off under reduced pressure and then the residue thus obtained was purified according to the silica gel chromatography (hexane: ethyl acetate = 6:1) to give a desired compound (4.54 g) as colorless powder. ¹H NMR (CDCl₃, 400 MHz): δ 4.18 (3H, s), 6.16 (1H, d, J = 8.6 Hz), 6.93 (1H, s), 7.43 (1H, d, J = 8.6 Hz).

### Example 6: Ethyl 4-bromo-2-ethyl-7-methoxypyrazolo[1,5-a]pyridine-3-carboxylate:

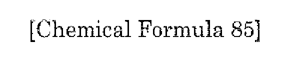

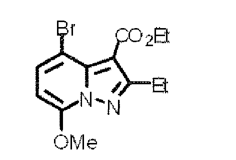

The same procedures used in Example 3 were carried out except for using the compound disclosed in Example 12 of WO 2006/095666 and ethyl 2-pentynoate to give a desired compound as yellow solid.
¹H NMR (CDCl_{3,} 400 MHz): δ 1.34 (3H, t, J = 7.6 Hz), 1.42 (3H, t, J = 7.3 Hz), 3.03 (2H, q, J = 7.3 Hz), 4.15 (3H, s), 4.41 (2H, q, J = 7.6 H), 6.09 (1H, d, J = 7.9 Hz), 7.50 (1H, d, J= 8.6 Hz).
EIMS (+) : 326 [M]⁺.

### Example 7: Benzyl 4-bromo-2-cyclopropyl-7-methoxypyrazolo[1,5-a] pyridine-3-carboxylate:

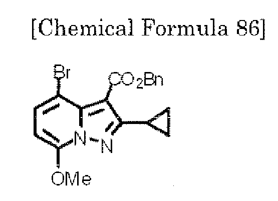

The same procedures as used in Example 3 were carried out except for using the compound disclosed in Example 12 of WO 2006/095666 and benzyl 3-cyclopropylpropionate to give a desired compound as a brown oily product.
¹H NMR (CDCl₃, 400 MHz): δ 0.94-0.99 (2H, m), 1.07-1.11 (2H, m), 2.35-2.41 (1H, m), 4.11 (3H, s), 5.42 (2H, s), 6.05 (1H, d, J = 7.9 Hz), 7.31-7.40 (3H, m), 7.44-7.50 (3H, m). CIMS (+): 401 [M+H]⁺.

### Example 8: 4-Bromo-2-ethyl-7-methoxypyrazolo[1,5-a]pyridine

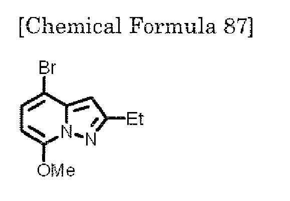

The compound (3.53 g) prepared in Example 6 was dissolved in a mixed solvent comprising dioxane (12.0 mL), methanol (6 mL) and water (9 mL), potassium hydroxide (3.64 g) was added to the resultant solution and then the resultant mixture was stirred for 24 hours under heat refluxing condition. After washing the reaction solution with ethyl acetate, the pH value of the aqueous layer was adjusted to 2 to 3 by the addition of a 1 mol/L hydrochloric acid and the aqueous layer was extracted three times with ethyl acetate. The combined extracts were dried over anhydrous sodium sulfate and the solvent was distilled off under reduced pressure to give a carboxylic acid (2.33 g) as black solid. The carboxylic acid (2.63 g) was dissolved in ethanol (44 mL), concentrated sulfuric acid (2.20 mL) was added to the resultant solution and then the resultant mixture was stirred for 4.5 hours under heat refluxing condition. After the pH value of the reaction solution was adjusted to 7 by the addition of a 10% sodium hydroxide aqueous solution, the solvent was distilled off and the pH of the residue was adjusted to 8 to 9 using a 10% sodium hydroxide aqueous solution. The aqueous solution was extracted three times with ethyl acetate, the extracted layer was washed, in order, with a saturated sodium hydrogen carbonate aqueous solution and saturated brine and then dried over anhydrous sodium sulfate. The solvent was distilled off under reduced pressure, then the resultant residue was purified by the aminated silica gel column chromatography (ethyl acetate) and then by the silica gel column chromatography (ethyl acetate: hexane = 1:2) to give a desired compound (911 mg) as pale yellow solid.
¹H NMR (CDCl₃, 400 MHz): δ 1.36 (3H, t, J = 7.3 Hz), 2.90 (2H, q, J = 7.3 Hz), 4.12 (3H, s), 5.92 (1H, d, J = 8.0 Hz), 6.44 (1H, s), 7.24 (1H, d, J = 8.0 Hz).
EIMS (+): 254 [M]+.

### Example 9: 4-Bromo-2-cyclopropyl-7-methoxypyrazolo[1,5-a]pyridine

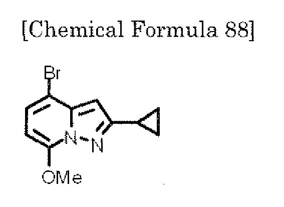

The same procedures as used in Example 8 were carried out except for using the compound prepared in Example 7 to give a desired compound as pale yellow solid.
¹H NMR (CDCl₃, 400 MHz): δ 0.86-0.90 (2H, m), 1.02-1.06 (2H, m), 2.18-2.22 (1H, m), 4.12 (3H, s), 5.90 (1H, d, J = 7.9 Hz), 6.20 (1H, s), 7.23 (1H, d, J = 7.9 Hz).
EIMS (+) : 266 [M]⁺.

### Example 10: 8-Bromo-5-methoxy-2-trifluoromethyl[1,2,4]triazolo[1,5-a]pyridine:

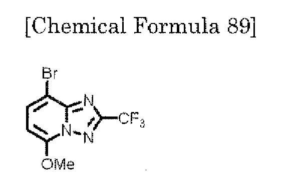

The compound (13.0 g) prepared in Example 1 was dissolved in methanol (100 mL), there were added, to the resultant solution, a mixture of triethylamine (13.0 mL), trifuloroacetic anhydride (6.6 mL) and methanol (20 mL) and the resultant mixture was stirred at ordinary temperature for 17.5 hours. Water was added to the reaction solution, the resultant mixture was extracted three times with ethyl acetate, the combined extracts were washed with saturated brine and then dried over anhydrous sodium sulfate. The solvent was distilled off under reduced pressure and then the resultant residue was purified by the silica gel column chromatography (hexane: ethyl acetate = 1:1) to give a desired compound (6.73 g) as brown powder.
¹ H NMR (CDCl₃, 400 MHz): δ 4.23 (3H, s), 6.41 (1H, d, J = 7.9 Hz), 7.87 (1H, d, J = 7.9 Hz).
EIMS (+): 295 [M]⁺.

### Example 11: 7-Bromo-4-methoxy-2-trifluoromethylbenzothiazole:

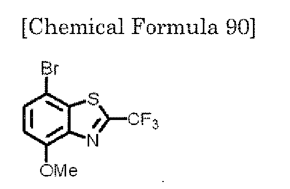

To a solution of 4-methoxy-2-trifluoromethylbenzothiazole (3.45 g) in acetic acid (25 mL), there was dropwise added a 1 mol/L solution of bromine in acetic acid (15.6 mL) at ordinary temperature and then the resultant mixture was stirred, with heating, at 75 °C for 6 hours. After the acetic acid was distilled off under reduced pressure, the residue was dissolved in ethyl acetate, the solution was washed with a saturated sodium hydrogen carbonate aqueous solution and then dried over anhydrous magnesium sulfate. The solution was concentrated by the distillation of the solvent under reduced pressure and then the resultant residue was purified by the silica gel column chromatography (hexane: ethyl acetate = 9:1) to give a desired compound (8.43 g) as colorless powder.
¹ H NMR (200 MHz, CDCl₃): δ 4.06 (3H, s), 6.91 (1H, d, J = 8.5 Hz), 7.59 (1H, d, J = 8.5 Hz).

### Example 12: 3-Bromo-2-hydroxymethyl-6-methoxyphenol:

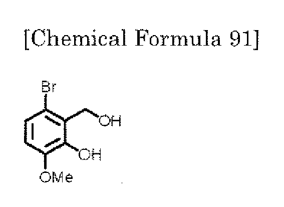

6-Bromo-2-hydroxy-3-methoxybenzaldehyde (1.00 g) was dissolved in methanol (30 mL), and then sodium borohydride (164 mg) was added to the resultant solution with stirring under ice-cooled condition. After stirring the mixture at ordinary temperature for 4 hours, a dilute hydrochloric acid solution was added to the mixture, which was extracted with ethyl acetate. After washing the extract with water and saturated brine, it was dried over anhydrous sodium sulfate and then the solvent was distilled off under reduced pressure to give a desired compound (911 mg) as pale yellow powder.
¹H NMR (CDCl₃, 400 MHz): δ 3.39 (3H, s), 4.91 (2H, s), 6.27 (1H, s), 6.70 (1H, d, J = 8.6 Hz), 7.07 (1H, d, J = 8.6 Hz).

### Example 13: (6-Bromo-2-hydroxy-3-methoxyphenyl)methyltriphenylphosphonium bromide:

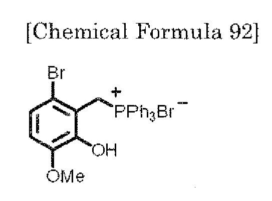

The compound (910 mg) prepared in Example 12 was dissolved in acetonitrile (10 mL), triphenylphosphonium bromide (1.47 g) was added to the resultant solution and then the resultant mixture was refluxed with heating for 5 hours. The half of the solvent of the mixture was distilled off under reduced pressure, ethyl acetate (50 mL) was added to the remainder, the crystals thus precipitated were collected by filtration and then dried to give a desired compound (2.20 g) as pale yellow powder.
¹H NMR (DMSO-d₆, 400 MHz): δ 3.63 (3H, s), 4.81 (2H, d, J = 14.1 Hz), 6.81. (1H, dd, J = 8.6, 1.8 Hz), 6.90 (1H, dd, J = 8.6, 0.6 Hz), 7.52-7.72 (12H, m), 7.80-7.84 (3H, m), 9.80 (1H, s).

### Example 14: 4-Bromo-7-methoxy-2-trifluoromethylbenzofuran:

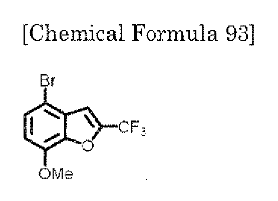

The compound (2.20 g) prepared in Example 13 was suspended in toluene (20 mL) under an argon gas atmosphere, followed by the addition of trifuloroacetic anhydride (0.612 mL) and triethylamine (1.64 mL) and the reflux, with heating, of the mixture for 5 hours. Water was added to the reaction mixture, the mixture was extracted with ethyl acetate, the extracted layer was washed with saturated brine and then dried over anhydrous sodium sulfate and the solvent was then distilled off under reduced pressure. The resultant residue was purified by the silica gel column chromatography (hexane: ethyl acetate = 10:1) to give a desired compound (1.01 g) as pale yellow powder.
¹H NMR (CDCl₃, 400 MHz): δ 4.01 (3H, s), 6.82 (1H, d, J = 8.6 Hz), 7.20-7.21 (1H, m), 7.38 (1H, d, J = 8.6 Hz).

### Example 15: O-(3-Bromo-2-formyl-6-methoxy)phenyl dimethylthiocarbamate:

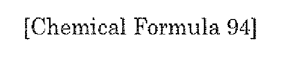

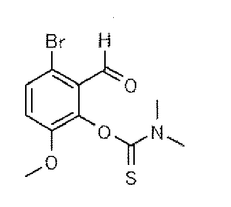

To a solution of 6-bromo-2-hydroxy-3-methoxybenzaldehyde (231 mg) in DMF (4.0 mL), added were triethylenediamine (224 mg) and dimethylthiocarbamoyl chloride (247 mg) and the resultant mixture was stirred at ordinary temperature for 12 hours. After the solvent was distilled off under reduced pressure, water was added to the resultant residue, followed by the extraction of the mixture with ethyl acetate. The extracted layer was dried over anhydrous magnesium sulfate and the residue obtained after the removal of the solvent through distillation was washed with diisopropyl ether to give a desired compound (258 mg) as pale yellow powder.
¹H NMR (CDCl₃, 400 MHz): δ 3.40 (3H, s), 3.45 (3H, s), 3.86 (3H, s), 7.05 (1H, d, J = 8.6 Hz), 7.51 (1H, d, J = 8.6 Hz), 10.20 (1H, s).
EIMS (+): 317 [M]⁺.

### Example 16: S-(3-Bromo-2-formyl-6-methoxy)phenyl dimethylthiocarbamate:

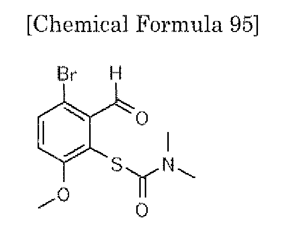

A solution of the compound (5.78 g) of Example 15 in diphenyl ether (57 mL) was stirred at 200°C for 30 minutes. The reaction mixture was cooled and then purified by the silica gel column chromatography (hexane: ethyl acetate = 1:1) to give a desired compound (3.28 g) as pale brown powder.
¹H NMR (CDCl₃, 400 MHz): δ 3.00 (3H, brs), 3.16 (3H, brs), 3.89 (3H, s), 6.97 (1H, d, J = 9.2 Hz), 7.64 (1H, d, J = 9.2 Hz), 10.25 (1H, s).
EIMS (+): 317 [M]⁺.

### Example 17: (6-Bromo-2-mercapto-3-methoxy)phenyl methanol:

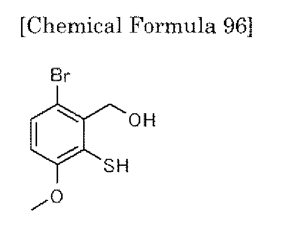

The compound (2.44 g) of Example 16 was suspended in isopropyl alcohol (60 mL), a 1 mol/L aqueous solution of sodium hydroxide (15.3 mL) was added to the suspension and the mixture was stirred at 60°C for 30 minutes. The solvent was evaporated under reduced pressure to concentrate the mixture, which was acidified by the addition of a 5% hydrochloric acid solution and then extracted with ethyl acetate. The extracted layer was washed with saturated brine and dried over anhydrous magnesium sulfate and then the solvent was distilled off under reduced pressure. The residue obtained was dissolved in methanol (60 mL), sodium borohydride (580 mg) was added to the resultant solution while ice-cooling the solution and then the resultant mixture was stirred at ordinary temperature for 30 minutes. The solvent was evaporated under reduced pressure to concentrate the mixture, which was acidified by the addition of a 5% hydrochloric acid solution and then extracted with ethyl acetate. The extracted layer was washed with saturated brine, dried over anhydrous magnesium sulfate and then the solvent was distilled off under reduced pressure to give a desired compound (1.95 g) as a pale purple-colored oily product.
¹H NMR (CDCl₃, 400 MHz): δ 1.99 (1H, brs), 3.90 (3H, s), 4.46 (1H, s), 4.93 (2H, s), 6.71 (1H, d, J = 8.6 Hz), 7.33 (1H, d, J = 8.6 Hz).
ELMS (+): 248 [M]⁺.

### Example 18: 4-Broma-7-methoxy-2-trifluoromethylbenzo[b]thiophone:

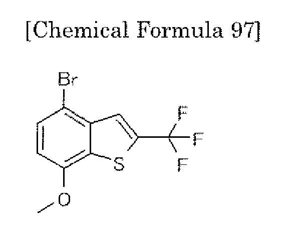

The compound (1.95 g) of Example 17 was dissolved in acetonitrile (15 mL), triphenylphosphonium bromide (2.90 g) was added to the solution and the mixture was refluxed for 17 hours. The solvent was evaporated under reduced pressure to concentrate the mixture, which was then washed with ethyl acetate to give colorless powder (4.39 g). To the resultant solid (4.35 g), added were toluene (60 mL), trifuloroacetic anhydride (1.18 mL) and triethylamine (3.17 mL) and the resultant mixture was refluxed for 3 hours. Water was added to the reaction mixture, the mixture was extracted with ethyl acetate, the extract was then washed with saturated brine and dried over anhydrous magnesium sulfate and the solvent was distilled off under reduced pressure. The residues thus obtained was purified by the silica gel column chromatography (hexane: ethyl acetate = 10:1) to give a desired compound (2.00 g) as colorless powder.
¹H NMR (CDCl₃, 400 MHz): δ 4.00 (3H, s), 6.75 (1H, d, J = 8.6 Hz), 7.53 (1H, d, J = 8.6 Hz), 7.79 (1H, q, J = 1.2 Hz).
EIMS (+): 310 [M]⁺.

### Example 19: 8-Methoxy-2-trifluoromethylimidazo[1,2-a]pyridine:

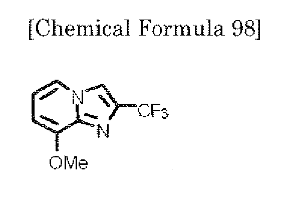

3-Bromo-1,1,1-trifluoropropan-2-one (5.00 g) was added to a solution of 2-amino-3-methoxypyridine (2.17 g) in ethanol (50 mL) and the resultant mixture was stirred at 70°C for 8 hours. After the addition of a saturated aqueous sodium hydrogen carbonate solution (10 mL) to the reaction mixture, the solvent was distilled off under reduced pressure and then the resultant residue was extracted with ethyl acetate. The extracted layer was dried over anhydrous magnesium sulfate, the solvent was distilled off under reduced pressure and the resultant residue was purified according to the silica gel column chromatography (hexane: ethyl acetate = 1:3) to give a desired compound (2.57 g) as white solid.
¹H NMR (CDCl₃, 400 MHz): δ 4.03 (3H, s), 6.55 (1H, d, J = 7.9 Hz), 6.82 (1H, dd, J = 7.9, 6.7 Hz), 7.78 (1H, d, J = 6.7 Hz), 7.86 (1H, s).
EIMS (+): 216 [M]⁺.

### Example 20: 5-Bromo-3-chloro-8-methoxy-2-trifluoromethylimidazo[1,2-a]pyridine:

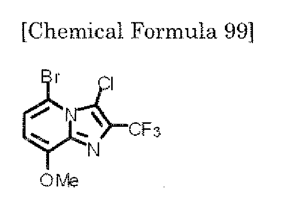

NCS (618 mg) was added to a solution of the compound of Example 19 (1.00 g) in DMF (15 mL) and the resultant mixture was stirred at 70°C for 30 minutes. The temperature of the reaction mixture was cooled to ordinary temperature, NBS (823 mg) was then added to the mixture and the mixture was stirred at 70°C for one hour. A saturated sodium hydrogen carbonate aqueous solution was added to the reaction mixture, the mixture was extracted with ethyl acetate, the extracted layer was washed, in order, with water and saturated brine and then dried over anhydrous magnesium sulfate. The residues obtained after the solvent was distilled off was purified according to the silica gel column chromatography (hexane: ethyl acetate = 3:2) to give a desired compound (1.26 g) as pale yellow powder.
¹H NMR (CDCl₃, 400 MHz): δ 4.02 (3H, s), 6.46 (1H, d, J = 7.9 Hz), 7.05 (1H, d, J = 7.9 Hz).
CIMS (+): 330 [M+H]⁺.

### Example 21: 4-Bromo-N-methyl-2-trifluoromethylpyrazolo[1,5-a]pyridine-7-amine:

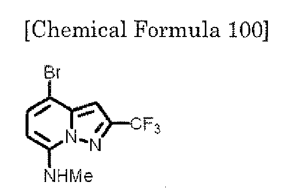

Potassium t-butoxide (1.14 g) was dissolved in DMSO (15 mL) under an argon gas atmosphere, N-methylformamide (0.596 mL) was added to the solution and the mixture was stirred at ordinary temperature for one hour. A solution of the compound of Example 5 (1.00 g) in DMSO (10 mL) was added at ordinary temperature and the resultant mixture was stirred for additional 50 minutes. Ice-water was added to the reaction mixture, the insoluble material precipitated out of the mixture was collected by filtration and washed with water to give a desired compound (832 mg) as colorless powder.
¹H NMR (CDCl₃, 400 MHz): δ 3.08 (3H, d, J = 5.5 Hz), 5.87 (1H, d, J = 8.6 Hz), 5.92 (1H, brs), 6.81 (1H, s), 7.39 (1H, d, J = 8.6 Hz).
EIMS (+): 293 [M]⁺.

### Example 22: 4-Bromo-3-chloro-7-methoxy-2-trifluoromethylpyrazolo[1,5-a]pyridine:

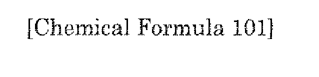

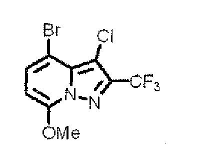

The compound of Example 5 (1.50 g) was dissolved in DMF (50 mL), NCS (1.02 g) was added to the resultant solution and the resultant mixture was stirred at 60°C for 3.5 hours. Water was added to the reaction mixture and the solid precipitated out of the mixture was collected by filtration. The resultant solid was purified according to the silica gel column chromatography (hexane: ethyl acetate = 4:1) to give a desired compound (825 mg) as colorless powder.
¹H NMR (CDCl₃, 400 MHz): δ 4.17 (3H, s), 6.14 (1H, d, J = 7.9 Hz), 7.46 (1H, d, J = 7.9 Hz).
EIMS (+): 328 [M]⁺.

### Example 23: 4-Bromo-3-chloro-N-methyl-2-trifluoromethylpyrazolo[1,5-a]pyridine-7-amine:

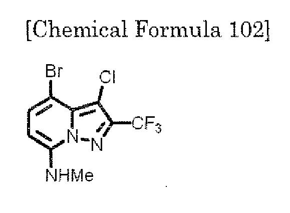

Potassium t-butoxide (842 mg) was dissolved in DMSO (13 mL) under an argon gas atmosphere, N-methylformamide (0.438 mL) was added to the resultant solution and the resultant mixture was stirred at ordinary temperature for one hour. A solution of the compound of Example 22 (825 mg) in DMSO (10 mL) was added to the mixture at ordinary temperature and the mixture was stirred for additional 35 minutes. Ice-water was added to the reaction mixture, the insoluble material precipitated out of the mixture was collected by filtration to give a desired compound (761 mg) as colorless powder.
¹H NMR (CDCl₃, 400 MHz): δ 3.08 (3H, d, J = 4.9 Hz), 5.87 (1H, d, J = 7.9 Hz), 5.99 (1H, brs), 7.43 (1H, d, J = 7.9 Hz).
EIMS (+): 327 [M]⁺.

### Example 24: 5-Bromo-8-methoxyquinazoline:

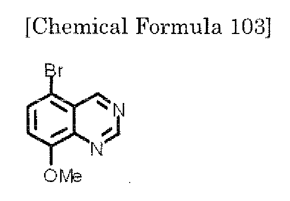

NBS (172 mg) was added to a solution of 8-methoxyquinazoline (155 mg) in DMF (3 mL) and the resultant mixture was allowed to stand at ordinary temperature for 4 days. A 5% aqueous solution of sodium hydrogen carbonate was added to the reaction mixture, the mixture was extracted with ethyl acetate, the extracted layer was washed, in order, with water and saturated brine and then the layer was dried over anhydrous magnesium sulfate. The residue obtained after the solvent was distilled off under reduced pressure was purified according to the silica gel column chromatography (ethyl acetate) to give a desired compound (181 mg) as a white solid.
¹H NMR (CDCl₃, 400 MHz): δ 4.10 (3H, s), 7.13 (1H, d, J = 8.6 Hz), 7.80 (1H, d, J = 8.6 Hz), 9.41 (1H, s), 9.69 (1H, s).
EIMS (+): 238 [M]⁺.

### Example 25: 2-Ethyl-4-methoxy-7-(4,4,5,5-tetramethyl[1,3,2]dioxaborolan-2-yl) benzoxazole:

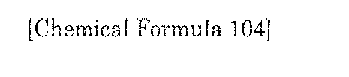

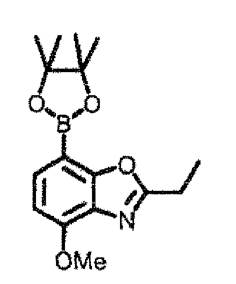

A solution of 7-bromo-2-ethyl-4-methoxybenzoxazole (100 mg), bis(pinacolato) diboron (109 mg), potassium 2-ethylhexanoate (85.4 mg) and 1,1-bis(diphenylphosphino)ferrocene palladium bichloride dichloromethane complex (15.9 mg) in 1,4-dioxane (2.0 mL) was stirred at 80°C for 1.5 hours under an argon gas atmosphere. After allowing the solution to cool, water was added to the reaction mixture, the resultant mixture was extracted with ethyl acetate, the extracted layer was dried over anhydrous sodium sulfate and then the solvent was distilled off. The residue thus obtained was purified by the silica gel column chromatography (hexane: ethyl acetate = 3.5:1) to obtain the desired compound (71.2 mg) as a colorless liquid.
1H NMR (CDCl₃, 400 MHz): δ 1.37 (12H, s), 1.46 (3H, t, J = 7.6 Hz), 3.00 (2H, q, J = 7.6 Hz), 4.03 (3H, s), 6.77 (1H, d, J = 8.6 Hz), 7.67 (1H, d, J = 8.6 Hz).
EIMS (+): 303 [M]⁺.

### Example 26: 7-Methoxy-4-(4,4,5,5-tetramethyl[1,3,2]dioxaborolan-2-yl)-2-trifluoromethylpyrazolo[1,5-a]pyridine:

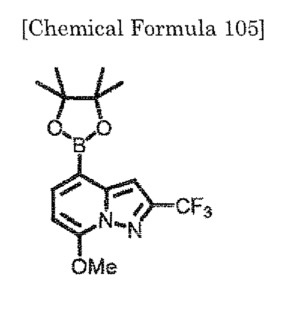

The compound (300 mg) prepared in Example 5 was dissolved in dioxane (10 mL) under an argon gas atmosphere, bis(pinacolato) diboron (284 mg), 1,1-bis(diphenylphosphino)ferrocene palladium dichloride dichloromethane complex (83.3 mg) and potassium 2-ethylhexanoate (279 mg) were added to the resultant solution, and the resultant mixture was stirred at 80°C for 3 hours. After the solvent of the reaction mixture was distilled off under reduced pressure, the resultant residue was purified by the silica gel column chromatography (hexane: ethyl acetate = 3:1) to obtain the desired compound (300 mg) as colorless powder.
1H NMR (DMSO-d₆, 400 MHz): δ 1.33 (12H, s), 4.16 (3H, s), 6.66 (1H, d, J = 7.9 Hz), 7.07 (1H, s), 7.77 (1H, d, J = 7.9 Hz).

### Example 27: 6-Bromo-2-hydroxy-3-methoxybenzamide:

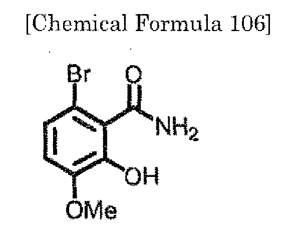

Methyl 6-bromo-2-hydroxy-3-methoxybenzoate (8.30 g) was dissolved in aqueous ammonia (100 mL), and the resultant solution was allowed to stand within a sealed tube at 70°C for 8 hours. The resultant precipitates were collected by filtration and dissolved in water, the pH of the resultant solution was adjusted to 2 to 3 by the addition of a 1 mol/L hydrochloric acid solution and then the solution was extracted twice with ethyl acetate. The combined extracts were dried over anhydrous sodium sulfate. The solvent was then distilled off under reduced pressure to give a desired compound (4.34 g) as white solid.
1H NMR (DMSO-d₆, 400 MHz): δ 3.78 (3H, s), 6.87 (1H, d, J = 9.2 Hz), 6.95-6.98 (1H, m), 7.40 (2H, s), 9.20 (1H, s).
EIMS (+): 245 [M]⁺.

### Example 28: 5-Bromo-3-(hydroxymethyl)-8-methoxy-2H-benzo[e][1,3]oxazin-4(3H)-one:

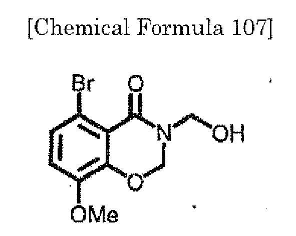

The compound (200 mg) prepared in Example 27 was dissolved in a mixed solvent comprising a 37% aqueous formalin solution (1.0 mL) and formic acid (1.0 mL) and then the solution was stirred for 5 hours under heat refluxing condition. The reaction mixture was poured into ice-water, the mixture was neutralized by the addition of a saturated aqueous sodium carbonate solution and the neutralized reaction mixture was extracted three times with ethyl acetate. The combined extracted layers were dried over anhydrous sodium sulfate. The solvent was distilled off under reduced pressure and then the resultant residue was purified by the silica gel column chromatography (ethyl acetate: hexane = 2:1) to obtain a desired compound (125 mg) as white solid.
1H NMR (CDCl₃, 400 MHz): δ 3.64 (1H, brt, J = 5.5 Hz), 3.89 (3H, s), 5.08 (2H, d, J = 5.5 Hz), 5.36 (2H, s), 6.87 (1H, d, J = 8.6 Hz), 7.30 (1H, d, J = 8.6 Hz).
EIMS(+): 287 [M]⁺.

### Example 29: 5-Bromo-8-methoxy-2H-benzo[e][1,3]oxazin-4(3H)-one

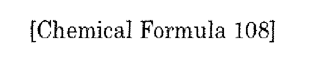

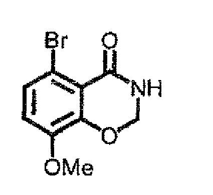

The compound (1.40 g) of Example 28 was dissolved in toluene (48.0 mL) and then the resultant solution was stirred for 1.5 hours under heat refluxing condition. After the solvent was distilled off under reduced pressure, the resultant residue was washed with diisopropyl ether to give a desired compound (1.15 g) as white solid.
1H NMR (CDCl₃, 400 MHz): δ 3.80 (3H, s), 5.07-5.09 (2H, m), 7.09 (1H, d, J = 8.6 Hz), 7.28 (1H, d, J = 8.6 Hz), 8.87 (1H, brt, J = 4.5 Hz).

### Example 30: 4-Bromo-7-methoxybenzo[d]oxazol-2(3H)-one:

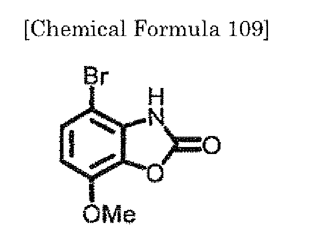

6-Bromo-2-hydroxy-3-methoxybenzoic acid (3.00 g) was dissolved in toluene (120 mL) under an argon gas atmosphere, followed by the addition of diphenylphosphoryl azide (2.89 mL) and triethylamine (1.97 mL) to the resultant solution at ordinary temperature and the stirring of the mixture at 80°C for 5 hours. The solvent was distilled off under reduced pressure and the resultant residue was then washed with ethyl acetate to give a desired compound (597 mg) as white solid.
1H NMR (DMSO-d₆, 400 MHz): δ 3.87 (3H, s), 6.79 (1H, d, J = 9.2 Hz), 7.25 (1H, d, J = 9.2 Hz), 12.10 (1H, s).
EIMS (+): 243 [M]⁺.

### Example 31: 5-Bromo-8-methoxy-2H-benzo[b][1,4]oxazin-3(4H)-one:

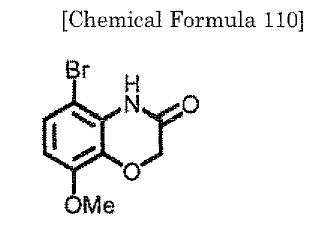

8-Methoxy-2H-benzo[b][1,4]oxazin-3(4H)-one (100 mg) was dissolved in DMF (5.5 mL) under an argon gas atmosphere, NBS (119 mg) was added to the solution at ordinary temperature and the resultant mixture was stirred at that temperature for 1.5 hours. A saturated aqueous sodium thiosulfate solution was added to the reaction mixture, which was extracted twice with ethyl acetate. The combined extracts were washed, in order, with water and saturated brine and then dried over anhydrous sodium sulfate. After the solvent was distilled off under reduced pressure, the residue thus obtained was purified by the silica gel column chromatography (ethyl acetate: hexane = 1:2) to obtain the desired compound (59.8 mg) as white solid.
1H NMR (CDCl₃, 400 MHz): δ 3.09 (3H, s), 4.66 (2H, s), 6.57 (1H, d, J = 9.2 Hz), 7.14 (1H, d, J = 9.2 Hz), 7.79 (1H, brs).
EIMS (+): 257 [M]⁺.

### Example 32: 2-(7-Methoxy-2-tnfluoromethylbenzo[b]thiophen-4-yl)-4,4,5,5-tetra methyl-1,3,2-dioxaborolane:

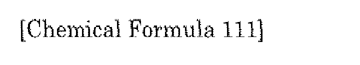

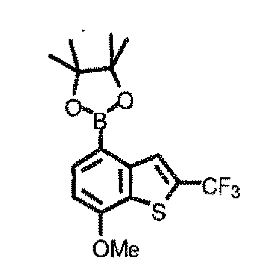

The same procedures as used in Example 25 were carried out except for using the compound prepared in Example 18 to give a desired compound as colorless powder.
1H NMR (CDCl₃, 400 MHz): δ 1.39 (12H, s), 4.03 (3H, s), 6.87 (1H, d, J = 8.0 Hz), 7.93 (1H, d, J = 8.0 Hz), 8.31 (1H, d, J = 1.2 Hz).

### Example 33: 7-Amino-4-bromo-2-trifluoromethylpyrazolo[1,5-a]pyridine:

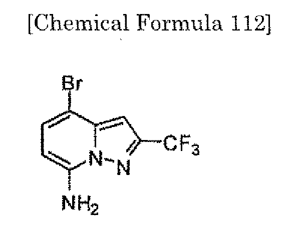

Potassium t-butoxide (570 mg) was dissolved in DMSO (10 mL) under an argon gas atmosphere, formamide (0.202 mL) was added to the resultant solution and the resultant mixture was stirred at ordinary temperature for one hour. A solution of the compound (500 mg) prepared in Example 5 in DMSO (5.0 mL) was added to the mixture, followed by the stirring of the resultant mixture at 60°C for 2.5 hours. A saturated aqueous ammonium chloride solution was added to the reaction mixture, which was extracted three times with ethyl acetate. The combined organic layers were washed with saturated brine and dried over anhydrous sodium sulfate and the solvent was distilled off under reduced pressure. The resultant residue was dissolved in methanol (10 mL), a solution of sodium hydroxide (203 mg) in water (5.0 mL) was added to the solution and the mixture was stirred at ordinary temperature for 2.5 hours. Water was added to the reaction mixture and the mixture was extracted three times with ethyl acetate. The combined organic layers were washed with saturated brine and dried over anhydrous sodium sulfate and then the solvent was distilled off under reduced pressure. The resultant residue was purified by the silica gel column chromatography (hexane: ethyl acetate = 4:1) to obtain the desired compound (315 mg) as yellow powder.
1H NMR (CDCl₃, 400 MHz): δ 5.26 (2H, s), 6.10 (1H, d, J = 7.9 Hz), 6.84 (1H, s), 7.34 (1H, d, J = 7.9 Hz).
EIMS (+): 279 [M]⁺.

### Example 34: 2-Trifluoromethylimidazo[1,2-a]pyridine-8-amine:

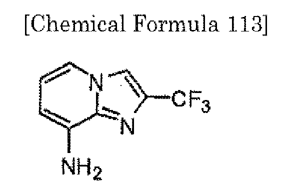

2,3-Diaminopyridine (14.1 g) was suspended in ethanol (260 mL), 3-bromo-1,1,1-trifluoropropan-2-one (24.7 g) was added to the resultant suspension and the resultant mixture was stirred for 8 hours under heat refluxing condition. After the solvent in the reaction mixture was distilled off under reduced pressure, a saturated aqueous sodium hydrogen carbonate solution was added to the resultant residue and the mixture was extracted three times with ethyl acetate. The combined organic layers were washed with saturated brine and dried over anhydrous sodium sulfate and then the solvent was distilled off under reduced pressure. The resultant residue was purified by the silica gel column chromatography (hexane: ethyl acetate = 3:1) to obtain the desired compound (15.7 g) as yellow powder.
1H NMR (CDCl₃, 400 MHz): δ 4.59 (2H, s), 6.39 (1H, dd, J = 7.3, 1.2 Hz), 6.72 (1H, t, J = 7.3 Hz), 7.59 (1H, d, J = 7.3 Hz), 7.81 (1H, d, J = 1.2 Hz).
EIMS (+) : 201 [M]⁺.

### Example 35: 5-Bromo-2-trifluoromethylimidazo[1,2-a]pyridine-8-amine:

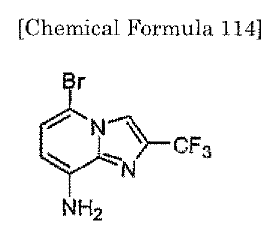

The compound (3.0 g) prepared in Example 34 was dissolved in DMF (100 mL), NBS (2.67 g) was added to this solution at 0°C and the mixture was stirred at 0°C for one hour. Water was added to the reaction mixture and the mixture was extracted three times with ethyl acetate. The combined organic layers were washed with saturated brine and dried over anhydrous sodium sulfate and then the solvent was distilled off under reduced pressure. The resultant residue was purified by the silica gel column chromatography (hexane: ethyl acetate = 4:1) to obtain the desired compound (1.96 g) as yellow powder.
1H NMR (CDCl₃, 400 MHz): δ 4.61 (2H, s), 6.38 (1H, d, J = 7.9 Hz), 6.93 (1H, d, J =7.9 Hz), 8.02 (1H, d, J = 1.2 Hz).
EIMS (+) : 279 [M]⁺.

### Example 36: t-Butyl 2-trifluoromethylimidazo[1,2-a]pyridin-8-yl carbamate:

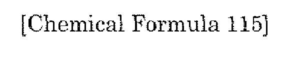

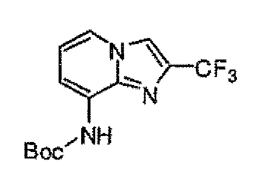

The compound (5.63 g) prepared in Example 34 was dissolved in THF (26 mL) under an argon gas atmosphere, a solution of sodium hexamethyldisilazane in THF (1.0 mol/L, 53.6 mL) was added to this solution and the resultant mixture was stirred at ordinary temperature for 30 minutes. A solution of t-butyl dicarbonate (5.85 g) in THF (26 mL) was slowly dropwise added to the mixture and then the resultant mixture was stirred at ordinary temperature for 3 hours. A saturated aqueous ammonium chloride solution was added to the reaction mixture and the mixture was extracted three times with ethyl acetate. The combined organic layers were washed with saturated brine and dried over anhydrous sodium sulfate and then the solvent was distilled off under reduced pressure. The resultant residue was purified by the silica gel column chromatography (hexane: ethyl acetate = 9:1) to obtain a desired compound (5.16 g) as yellow powder.
1H NMR (CDCl₃, 400 MHz): δ 1.54 (9H, s), 6.88 (1H, t, J = 7.3 Hz), 7.79 (1H, d, J = 7.3 Hz), 7.82 (1H, s), 7.86 (1H, s), 7.91 (1H, d, J = 7.3 Hz).
ESIMS (+): 301 [M+H]⁺.

### Example 37: t-Butyl 2-trifluoromethylimidazo[1,2-a]pyridin-8-yl(methyl) carbamate:

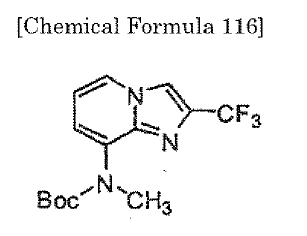

The compound (5.12 g) prepared in Example 36 was dissolved in DMF (100 mL) under an argon gas atmosphere, a 60% sodium hydride (884 mg) was added to the resultant solution at 0°C and the resultant mixture was stirred at ordinary temperature for 30 minutes. Methyl iodide (1.38 mL) was added to the mixture at 0°C and the resultant mixture was stirred at ordinary temperature for one hour. The reaction mixture was poured into ice-water and the precipitates thus formed were collected by filtration to obtain a desired compound (5.38 g) as colorless powder.
1H NMR (CDCl₃, 400 MHz): δ 1.41 (9H, s), 3.41 (3H, s), 6.88 (1H, t, J = 7.3 Hz), 7.20 (1H, d, J= 7.3 Hz), 7.90 (1H, s), 8.03 (1H, dd, J = 7.3, 1.2 Hz).
CIMS (+) : 316 [M+H]⁺.

### Example 38: N-Methyl-2-trifluoromethylimidazo[1,2-a]pyridine-8-amine:

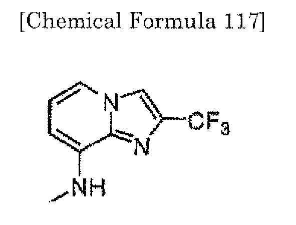

The compound (3.59 g) prepared in Example 37 was dissolved in dichloromethane (60 mL), trifluoroacetic acid (10 mL) was added to the resultant solution and the resultant mixture was stirred at ordinary temperature for 30 minutes. After the solvent of the reaction mixture was distilled off under reduced pressure, a saturated aqueous sodium hydrogen carbonate was added to the resultant residue and the mixture was extracted three times with ethyl acetate. The combined extracted layers were washed with saturated brine and dried over anhydrous sodium sulfate and then the solvent was distilled off under reduced pressure. The resultant residue was purified by the silica gel column chromatography (ethyl acetate) to obtain a desired compound (2.56 g) as green-colored powder.
1H NMR (CDCl₃, 400 MHz): δ 2.98 (3H, d, J = 4.9 Hz), 5.21 (1H, brs), 6.12 (1H, d, J = 7.3 Hz), 6.77 (1H, t, J = 7.3 Hz), 7.50 (1H, d, J = 7.3 Hz), 7.78 (1H, s).
EIMS (+): 215 [M]⁺.

### Example 39: 5-Bromo-N-methyl-2-trifluoromethylimidazo[1,2-a]pyridine-8-amine:

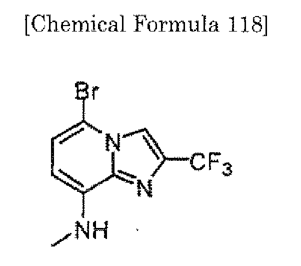

The compound (2.39 g) prepared in Example 38 was dissolved in DMF (80 mL), NBS (2.07 g) was added to the resultant solution at 0°C and the resultant mixture was stirred at 0°C for 25 minutes. Water was added to the reaction mixture and the mixture was extracted three times with ethyl acetate. The combined extracts were washed with saturated brine and dried over anhydrous sodium sulfate and then the solvent was distilled off under reduced pressure. The resultant residue was purified by the silica gel column chromatography (hexane: ethyl acetate = 20:1) to obtain a desired compound (2.60 g) as colorless powder.
1H NMR (CDCl₃, 400 MHz): δ 2.97 (3H, d, J = 4.9 Hz), 5.24 (1H, brs), 6.09 (1H, d, J = 7.9 Hz), 6.97 (1H, d, J = 7.9 Hz), 7.99 (1H, s).
EIMS (+): 293 [M]⁺.

### Example 40: t-Butyl 3-chloro-2-trifluoromethylimidazo[1,2-a]pyridin-8-yl (methyl) carbamate:

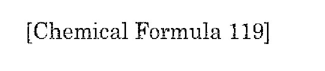

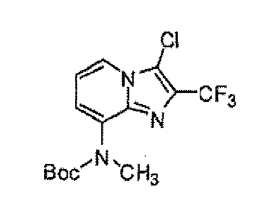

The compound (5.38 g) prepared in Example 37 was dissolved in DMF (100 mL), NCS (2.50 g) was added to this solution, and the resultant mixture was stirred at 60°C for one hour. Water was added to the reaction mixture and the mixture was extracted three times with ethyl acetate. The combined organic layers were washed with saturated brine and dried over anhydrous sodium sulfate and then the solvent was distilled off under reduced pressure. The resultant residue was purified by the silica gel column chromatography (hexane: ethyl acetate = 3:1) to obtain the desired compound (5.25 g) as yellow powder.
1H NMR (CDCl₃, 400 MHz): δ 1.42 (9H, s), 3.40 (3H, s), 7.03 (1H, t, J = 7.3 Hz), 7.27 (1H, d, J = 7.3 Hz), 8.03 (1H, dd, J = 7.3, 1.2 Hz).
CIMS(+): 350[M+H]⁺.

### Example 41: t-Butyl 5-bromo-3-chloro-2-trifluoromethylimidazo[1,2-a]pyridin-8-yl (methyl)carbamate:

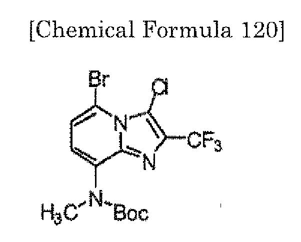

The compound (4.74 g) prepared in Example 40 was dissolved in DMF (100 mL), NCS (3.15 g) was added to the resultant solution, and the resultant mixture was stirred at 60°C for 1.5 hours. Water was added to the reaction mixture and the mixture was extracted three times with ethyl acetate. The combined organic layers were washed with saturated brine and dried over anhydrous sodium sulfate and then the solvent was distilled off under reduced pressure. The resultant residue was purified by the silica gel column chromatography (hexane: ethyl acetate = 4:1) to obtain the desired compound (5.32 g) as colorless powder.
1H NMR (CDCl₃, 400 MHz): δ 1.41 (9H, s), 3.34 (3H, s), 7.06 (1H, d, J = 7.9 Hz), 7.13 (1H, d, J = 7.9 Hz).
CIMS (+): 428 [M+H]⁺.

### Example 42: 6-Bromo-4-chloroisoquinoline:

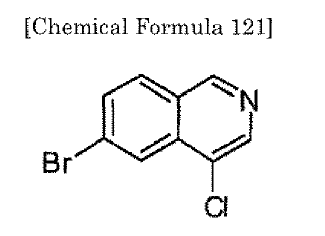

6-Bromoisoquinoline (Bioorg. Med. Chem. Lett. 2002, 12, 827) (200 mg) was dissolved in sulfuryl chloride (0.5 mL) and the resultant solution was stirred at 60°C for 5 minutes. Sulfuryl chloride (0.5 mL) was added to the resultant solution and the mixture was further stirred for 10 minutes. A saturated aqueous sodium hydrogen carbonate solution was added to this reaction mixture and the mixture was extracted three times with ethyl acetate. The combined organic layers were washed with saturated brine and dried over anhydrous sodium sulfate and then the solvent was distilled off under reduced pressure. The resultant residue was purified by the silica gel column chromatography (hexane: ethyl acetate = 2:1) to obtain the desired compound (119 mg) as colorless powder.
1H NMR (CDCl₃, 400 MHz): δ 7.78 (1H, dd, J = 8.6, 1.8 Hz), 7.88 (1H, d, J = 8.6 Hz), 8.39 (1H, d, J = 1.8 Hz), 8.61 (1H, s), 9.12 (1H, s).
EIMS (+): 241 [M]⁺.

### Example 43: 6-Bromo-5-chloroisoquinoline:

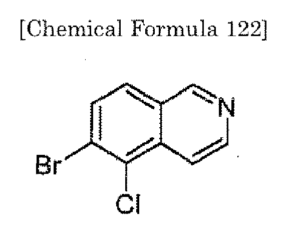

6-Bromoisoquinoline (200 mg) was dissolved in concentrated sulfuric acid (2.0 mL), NCS (257 mg) was added to the resultant solution at 0°C and the resultant mixture was stirred at 0°C for one hour. NCS (128 mg) was added to the mixture, the resultant mixture was stirred at 50°C for 5 hours, further NCS (128 mg) was added thereto and the mixture was stirred for 6 hours. The reaction mixture was poured into ice, the pH value of the mixture was adjusted to 14 by the addition of a 10% aqueous sodium hydroxide solution and the mixture was extracted three times with dichloromethane. The combined organic layers were washed with saturated brine and dried over anhydrous sodium sulfate and the solvent was distilled off under reduced pressure. The resultant residue was purified by the silica gel column chromatography (hexane: ethyl acetate = 2:1) to obtain a desired compound (208 mg) as colorless powder.
1H NMR (CDCl₃, 400 MHz): δ 7.78 (1H, d, J = 8.6 Hz), 7.82 (1H, d, J = 8.6 Hz), 8.05 (1H, d, J = 6.1 Hz), 8.68 (1H, d, J = 6.1 Hz), 9.25 (1H, s).
EIMS(+):241 [M]⁺.

### Example 44: 6-Bromoisoquinoline 2-oxide

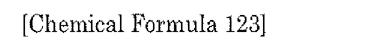

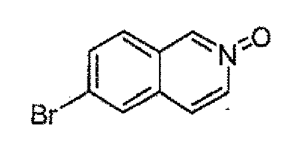

6-Bromoisoquinoline (200 mg) was dissolved in chloroform (10 mL), m-chloroperbenzoic acid (510 mg) was added to the resultant solution and the resultant mixture was stirred at ordinary temperature for one hour. A saturated aqueous sodium hydrogen carbonate solution and sodium thiosulfate pentahydrate (953 mg) were added to the reaction mixture, and then the resultant mixture was extracted three times with ethyl acetate. The combined organic layers were washed with saturated brine and dried over anhydrous sodium sulfate and the solvent was distilled off under reduced pressure. Then, the resultant residue was purified by the silica gel column chromatography (ethyl acetate: methanol = 20:1) to obtain the desired compound (224 mg) as colorless powder.
1H NMR (CDCl₃, 400 MHz): δ 7.60-7.61 (2H, m), 7.71 (1H, dd, J = 8.6, 1.5 Hz), 7.98 (1H, s), 8.16 (1H, dd, J = 7.3, 1.8 Hz), 8.74 (1H, s).
CIMS (+): 224 [M+H]⁺.

### Example 45: 6-(4,4,5,5-Tetramethyl-1,3,2-dioxaborolan-2-yl) isoquinoline:

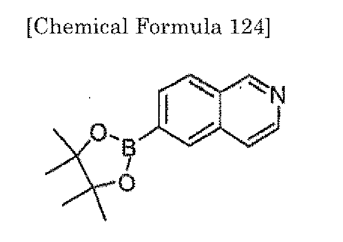

The same procedures as used in Example 25 were carried out except for using 6-bromoisoquinoline to give a desired compound as colorless powder.
1H NMR (CDCl₃, 400 MHz): δ 1.40 (12H, s), 7.68 (1H, d, J = 5.5 Hz), 7.96-7.96 (2H, m), 8.34 (1H, s), 8.54 (1H, d, J = 5.5 Hz), 9.27 (1H, s).
EIMS (+): 255 [M]⁺.

### Example 46: 4-Chloro-6-(4,4,5,5-tetramethyl-1,3,2-dioxaborolan-2-yl) isoquinoline:

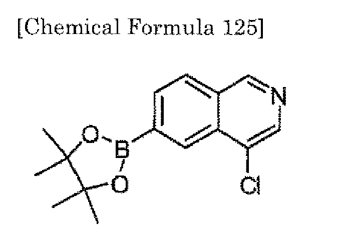

The same procedures as used in Example 25 were carried out except for using the compound prepared in Example 42 to give a desired compound as colorless powder.
1H NMR (CDCl₃, 400 MHz): δ 1.41 (12H, s), 7.98 (1H, d, J = 8.6 Hz), 8.05 (1H, d, J = 8.6 Hz), 8.59 (1H, s), 8.67 (1H, s), 9.16 (1H, s).
EIMS (+): 289 [M]⁺.

### Example 47: 6-(7-Methoxy-2-trifluoromethylpyrazolo[1,5-a]pyridin-4-yl) isoquinoline:

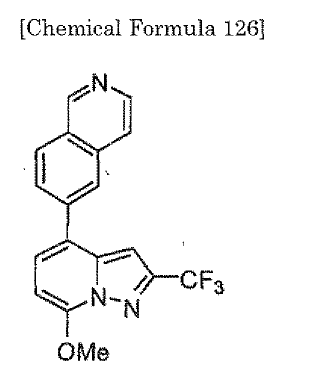

6-Bromoisoquinoline (209 mg), the compound (342 mg) prepared in Example 26 and tetrakis(triphenylphosphine) palladium (116 mg) were dissolved in 1,4-dioxane (5.0 mL) under an argon gas atmosphere, then a 2.0 mol/L aqueous sodium carbonate solution (1.05 mL) was added to the resultant solution and the resultant mixture was stirred for 2 hours under heat refluxing condition. Water was added to the reaction mixture and the resultant mixture was extracted three times with ethyl acetate. The combined organic layers were washed with saturated brine and dried over anhydrous sodium sulfate, the solvent was distilled off under reduced pressure and the resultant residue was purified by the silica gel column chromatography (ethyl acetate) and then washed with diisopropyl ether to obtain a desired compound (184 mg) as yellow powder.
1H NMR (CDCl₃, 400 MHz): δ 4.21 (3H, s), 6.82 (1H, d, J = 7.9 Hz), 7.32 (1H, s), 7.73 (1H, d, J = 7.9 Hz), 7.95 (1H, d, J = 5.5 Hz), 7.99 (1H, dd, J = 8.6, 1.2 Hz), 8.26 (1H, d, J = 8.6 Hz), 8.29 (1H, s), 8.55 (1H, d, J = 5.5 Hz), 9.37 (1H, s).
HREIMS (+): 343.0969 (Calculated for C₁₈H₁₂F₃N₃O: 343.0932).
Elemental Analysis: Found: C 62.60%, H 3.51%, N 11.88%; Calculated (for C₁₈H₁₂F₃N₃O): C 62.97%, H 3.52%, N 12.24%.

### Example 48: 6-(7-Methoxy-2-trifluoromethylpyrazolo[1,5-a]pyridin-4-yl) isoquinoline 2-oxide:

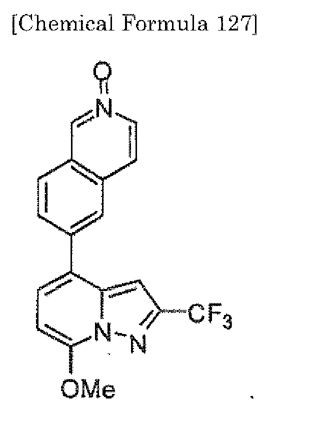

The same procedures as used in Example 47 were carried out except for using the compound of Example 44 and the compound of Example 26 to give a desired compound as yellow powder.
1H NMR (CDCl₃, 400 MHz): δ 4.26 (3H, s), 6.42 (1H, d, J = 7.9 Hz), 6.99 (1H, s), 7.41 (1H, d, J = 7.9 Hz), 7.75 (1H, d, J = 6.7 Hz), 7.87 (2H, s), 8.02 (1H, s), 8.21 (1H, d, J = 6.7 Hz), 8.82 (1H, s).
HRCIMS (+): 360.09821 (Calculated for C₁₈H₁₃F₃N₃O₂: 360.09599).

### Example 49: 4-Chloro-6-(7-methoxy-2-trifluoromethylpyrazolo[1,5-a]pyridin-4-yl) isoquinoline:

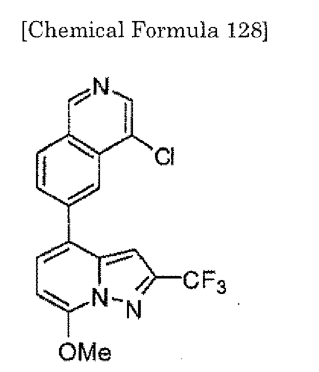

The same procedures as used in Example 47 were carried out except for using the compound of Example 42 and the compound of Example 26 to give a desired compound as colorless powder.
1H NMR (CDCl₃, 400 MHz): δ 4.27 (3H, s), 6.44 (1H, d, J = 7.9 Hz), 7.02 (1H, s), 7.48 (1H, d, J = 7.9 Hz), 7.95 (1H, dd, J = 8.6, 1.8 Hz), 8.15 (1H, d, J = 8.6 Hz), 8.41 (1H, s), 8.66 (1H, s), 9.22 (1H, s).
HRESIMS (+): 378.06302 (Calculated for C₁₈H₁₂ClF₃N₃O: 378.06210).
Elemental Analysis: Found: C 57.29%, H 3.11%, N 10.87%; Calculated (for C₁₈H₁₁ClF₃N₃O): C 57.23%, H 2.94%, N 11.12%.

### Example 50: 5-Chloro-6-(7-methoxy-2-trifluoromethylpyrazolo[1,5-a]pyridin-4-yl) isoquinoline:

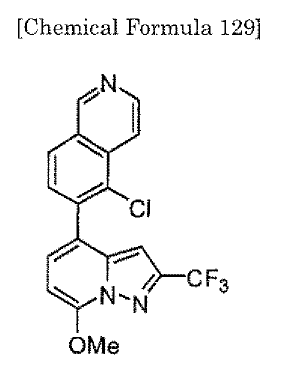

The same procedures as used in Example 47 were carried out except for using the compound of Example 43 and the compound of Example 26 to give a desired compound as colorless powder.
1H NMR (CDCl₃, 400 MHz): δ 4.27 (3H, s), 6.42 (1H, d, J = 7.9 Hz), 6.56 (1H, s), 7.37 (1H, d, J = 7.9 Hz), 7.65 (1H, d, J = 7.9 Hz), 8.03 (1H, d, J = 7.9 Hz), 8.16 (1H, d, J = 6.1 Hz), 8.74 (1H, d, J = 6.1 Hz), 9.35 (1H, s).
HRESIMS (+): 378.06192 (Calculated for C₁₈H₁₂ClF₃N₃O: 378.06210).
Elemental Analysis : Found: C 56.95%, H 3.00%, N 10.87%; Calculated (for C₁₈H₁₁ClF₃N₃O): C 57.23%, H 2.94%, N 11.12%.

### Example 51: 6-(3-Chloro-7-methoxy-2-trifluoromethylpyrazolo[1,5-a]pyridin-4-yl) isoquinoline:

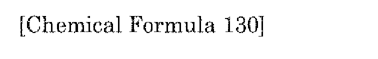

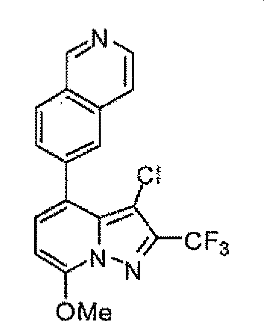

The same procedures as used in Example 47 were carried out except for using the compound of Example 45 and the compound of Example 22 to give the desired compound as colorless powder.
1H NMR (CDCl₃, 400 MHz): δ 4.22 (3H, s), 6.84 (1H, d, J = 7.9 Hz), 7.54 (1H, d, J = 7.9 Hz), 7.78 (1H, dd, J = 8.6, 1.8 Hz), 7.89 (1H, d, J = 6.1 Hz), 8.06 (1H, d, J = 1.8 Hz), 8.20 (1H, d, J = 8.6 Hz), 8.55 (1H, d, J = 6.1 Hz), 9.38 (1H, s).
HRESIMS (+): 378.06245 (Calculated for C₁₈H₁₂ClF₃N₃O: 378.06210).
Elemental Analysis : Found: C 57.03%, H 3.22%, N 10.78%; Calculated (for C₁₈H₁₁ClF₃N₃O): C 57.23%, H 2.94%, N 11.12%.

### Example 52: 4-Chloro-6-(3-chloro-7-methoxy-2-trifluoromethylpyrazolo[1,5-a] pyridin-4-yl) isoquinoline:

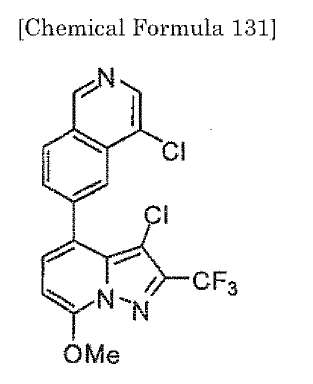

The same procedures as used in Example 47 were carried out except for using the compound of Example 46 and the compound of Example 22 to give a desired compound as yellow powder.
1H NMR (CDCl₃, 400 MHz): δ 4.26 (3H, s), 6.41 (1H, d, J = 7.9 Hz), 7.32 (1H, d, J = 7.9 Hz), 7.76 (1H, dd, J = 8.6, 1.5 Hz), 8.08 (1H, d, J = 8.6 Hz), 8.24 (1H, s), 8.66 (1H, s), 9.23 (1H, s).
HRESIMS (+): 411.0152 (Calculated for C₁₈H₁₀Cl₂F₃N₃O: 411.0153).

### Example 53: 6-(2-Cyclopropyl-7-methoxypyrazolo[1,5-a]pyridin-4-yl) isoquinoline:

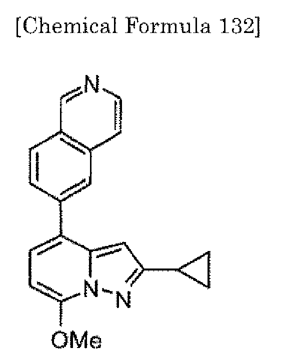

The same procedures as used in Example 47 were carried out except for using the compound of Example 45 and the compound of Example 9 to give a desired compound as yellow powder.
1H NMR (DMSO-d₆, 400 MHz): δ 0.85-0.86 (2H, m), 0.96-1.00 (2H, m), 2.08-2.10 (1H, m), 4.12 (3H, s), 6.45 (1H, d, J = 7.9 Hz), 6.59 (1H, s), 7.46 (1H, d, J = 7.9 Hz), 7.91 (1H, d, J = 6.1 Hz), 7.96 (1H, dd, J = 8.6, 1.8 Hz), 8.21 (2H, d, J = 8.6 Hz), 8.53 (1H, d, J = 6.1 Hz), 9.34 (1H, s).
HREIMS (+) : 316.14482 (Calculated for C₂₀H₁₈N₃O: 316.14499.
Elemental Analysis : Found: C 75.84%, H 5.57%, N 12.91%; Calculated (for C₂₀H₁₇N₃O · 1/10H₂O): C 75.74%, H 5.47%, N 13.25%.

### Example 54: 6-(2-Ethyl-7-methoxypyrazolo[1,5-a]pyridin-4-yl) isoquinoline:

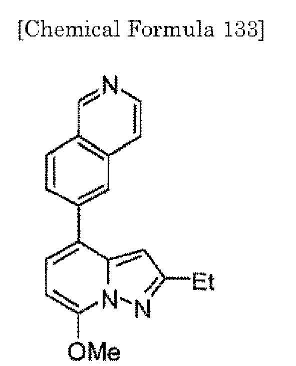

The same procedures as used in Example 47 were carried out except for using the compound of Example 45 and the compound of Example 8 to give a desired compound as yellow powder.
1H NMR (DMSO-d₆, 400 MHz): δ 1.27 (3H, t, J = 7.3 Hz), 2.79 (2H, q, J = 7.3 Hz), 4.13 (3H, s), 6.47 (1H, d, d = 7.9 Hz), 6.67 (1H, s), 7.47 (1H, d, J = 7.9 Hz), 7.91 (1H, d, J = 6.1 Hz), 7.97 (1H, dd, J = 8.6, 1.8 Hz), 8.22 (2H, d, J = 8.6 Hz), 8.53 (1H, d, J = 6.1 Hz), 9.34 (1H, s).
HRESIMS (+): 304.14534 (Calculated for C₁₉H₁₈N₃O: 304.14499.
Elemental Analysis : Found: C 75.16%, H 5.81%, N 13.55%; Calculated (for C₁₉H₁₇N₃O): C 75.22%, H 5.65%, N 13.85%.

### Example 55: 4-(Isoqumolm-6-yl)-N-methyl-2-trifluoromethylpyrazolo [1,5-a] pyridine-7-amine:

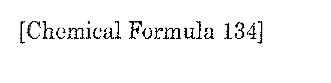

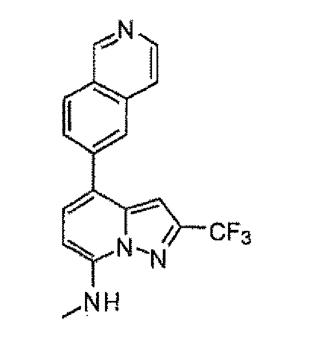

The same procedures as used in Example 47 were carried out except for using the compound of Example 45 and the compound of Example 21 to give a desired compound as yellow powder.
1H NMR (CDCl₃, 400 MHz): δ 3.18 (3H, d, J = 4.9 Hz), 6.15 (1H, d, J = 7.9 Hz), 6.14 (1H, brs), 6.99 (1H, s), 7.48 (1H, d, J = 7.9 Hz), 7.71 (1H, d, J = 6.1 Hz), 7.87 (1H, dd, J = 8.6, 1.8 Hz), 8.02 (1H, s), 8.07 (1H, d, J = 8.6 Hz), 8.57 (1H, d, J = 6.1 Hz), 9.29 (1H, s).
HREIMS (+): 342.1095 (Calculated for C₁₈F₁₃F₃N₄ : 342.1092).

### Example 56: 3-Chloro-4-(isoquinolin-6-yl)-N-methyl-2-trifluoromethylpyrazolo [1,5-a]pyridine-7-amine:

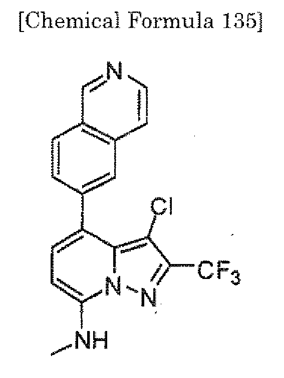

The same procedures as used in Example 47 were carried out except for using the compound of Example 45 and the compound of Example 23 to give a desired compound as yellow powder.
1H NMR (CDCl₃, 400 MHz): δ 3.02 (3H, d, J = 4.9 Hz), 6.33 (1H, d, J = 7.9 Hz), 7.49 (1H, d, J = 7.9 Hz), 7.55 (1H, q, J = 4.9 Hz), 7.75 (1H, dd, J = 8.6, 1.5 Hz), 7.87 (1H, d, J = 6.1 Hz), 8.00 (1H, s), 8.16 (1H, d, J = 8.6 Hz), 8.52 (1H, d, J = 6.1Hz), 9.35 (1H, s).
HREIMS (+): 376.0744 (Calculated for C₁₈H₁₂ClF₃N₄ : 376.0703).

### Example 57: 6-(3-Chloro-8-methoxy-2-trifluoromethylimidazo[1,2-a]pyridin-5-yl) isoquinoline:

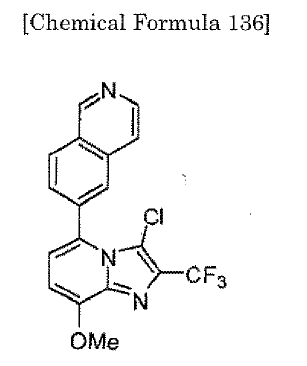

The same procedures as used in Example 47 were carried out except for using the compound of Example 45 and the compound of Example 22 to give a desired compound as white powder.
1H NMR (DMSO-d₆, 400 MHz): δ 4.02 (3H, s), 7.01 (1H, d, J = 7.9 Hz), 7.06 (1H, d, J = 7.9 Hz), 7.85 (1H, dd, J = 8.6, 1.5 Hz), 7.90 (1H, d, J = 6.1 Hz), 8.17 (1H, s), 8.19 (1H, d, J = 8.6 Hz), 8.58 (1H, d, J = 6.1 Hz).
HRESIMS (+): 378.06210 (Calculated for C₁₈H₁₂F₃N₃O: 378.06210).
Elemental Analysis : Found: C 56.55%, H 3.01%, N 10.98%; Calculated (for C₁₈H₁₁F₃N₃O · 1/5H₂O): C 56.69%, H 3.01%, N 11.02%.

### Example 58: 3-Chloro-5-(isoquinolin-6-yl)-N-methyl-2-trifluoromethylimidazo[1,2 a]pyridine-8-amine:

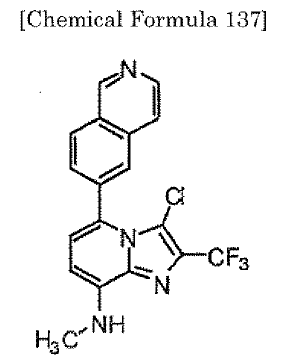

The compound (300 mg) prepared in Example 41 and the compound (179 mg) prepared in Example 45 were dissolved in 1,4-dioxane (7.0 mL) under an argon gas atmosphere, tetrakis(triphenylphosphine) palladium (80.9 mg) and a 2.0 mol/L aqueous sodium carbonate solution (1.4 mL) were then added to the resultant solution, and the resultant mixture was stirred at 100°C for 8 hours. Water was added to the reaction mixture and the resultant mixture was extracted three times with ethyl acetate. The combined organic layers were washed with saturated brine and dried over anhydrous sodium sulfate and the solvent was distilled off under reduced pressure. The resultant residue was purified by the silica gel column chromatography (hexane: ethyl acetate = 1:4), followed by the dissolution of the resultant amorphous substance in dichloromethane (5.0 mL), the addition of trifluoroacetic acid (2.0 mL) and the subsequent stirring of the resultant mixture at ordinary temperature for one hour. The solvent of the reaction mixture was distilled off under reduced pressure, a saturated aqueous sodium hydrogen carbonate solution was added to the residue thus obtained and then the mixture was extracted three times with ethyl acetate. The combined organic layers were washed with saturated brine and dried over anhydrous sodium sulfate and the solvent was distilled off under reduced pressure. The resultant residue was purified by the silica gel column chromatography (hexane: ethyl acetate = 1:1) and then washed with diisopropyl ether to obtain the desired compound as yellow powder.
1H NMR (DMSO-d₆, 400 MHz): δ 2.88 (3H, d, J = 4.9 Hz), 6.34 (1H, d, J = 7.9 Hz), 6.77 (1H, q, J = 4.9 Hz), 6.97 (1H, d, J = 7.9 Hz), 7.79 (1H, dd, J = 7.9, 1.8 Hz), 7.88 (1H, d, J = 5.5 Hz), 8.09 (1H, s), 8.15 (1H, d, J = 7.9 Hz), 8.55 (1H, d, J = 5.5 Hz), 9.37 (1H, s).
HRESIMS (+): 377.07770 (Calculated for C₁₈H₁₃ClF₃ N₄ : 377.07808).
Elemental Analysis: Found: C 57.22 %, H 3.21 %, N 14.80 %; Calculated (for C₁₈H₁₂ClF₃ N₄) : C 57.38 %, H 3.21 %, N 14.87 %.

### Example 59: 5-(Isoquinolin-6-yl)-8-methoxyquinoline:

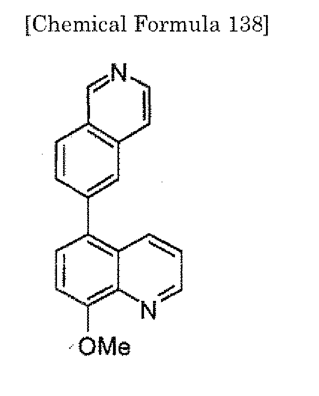

The same procedures as used in Example 47 were carried out except for using the compound of Example 45 and 5-bromo-8-methoxyquinoline to give a desired compound as yellow powder.
1H NMR (HMSO-d6, 400 MHz): δ 4.03 (3H, s), 7.33 (1H, d, J = 7.9 Hz), 7.55 (1H, dd, J = 8.6, 4.3 Hz), 7.62 (1H, d, J = 7.9 Hz), 7.78 (1H, dd, J = 8.6, 1.5 Hz), 7.90 (1H, d, J = 5.5 Hz), 8.06 (1H, s), 8.20 (1H ,dd, J = 8.6, 1.5 Hz), 8.25 (1H, d, J = 8.6 Hz), 8.56 (1H, d, J = 5.5 Hz), 8.90 (1H, dd, J = 4.3, 1.8 Hz).
HREIMS (+): 286.1137 (Calculated for C₁₉H₁₄N₂O: 286.1106).
Elemental Analysis: Found: C 79.29%, H 5.02%, N 9.61%; Calculated (for C₁₉H₁₄N₂O · 1/10H₂O): C 79.20%, H 4.97%, N 9.72%.

### Test Example 1: Phosphodiesterase-Inhibitory Activity:

Regarding the cDNAs of the PDE3A catalytic zone (hereunder referred to as "PDE4Acat") and the PDE4Bcat, the human-derived RNA was subjected to the RT-PCR technique to isolate the desired cDNAs of the "PDE4Acat" and "PDE4Bcat". Each cDNA fragment thus isolated was introduced into insect cells Sf9 using Gateway system (available from Invitrogen Company) and Bac-to-Bac™ Baculovirus Expression system (available from Invitrogen Company) to make the cells express the respective target PDE protein. These recombinant PDE4Acat and PDE4Bcat were obtained by subjecting the culture supernatants or cell-extracts of the Sf9 cells which were able to highly expressed these PDE proteins to the purification according to the ion-exchange chromatography technique and the resultant purified PDE4Acat and PDE4Bcat were used in the following experiments.

The test compound was stepwise diluted 4 times with a 15% DMSO solution, starting from a solution of the compound having an initial concentration of 4 mmol/L to thus prepare solutions thereof having a concentration ranging from 15 nmol/L to 4mmol/L (in the following experiments, the final concentration of the compound ranged from 1.5 nmol/L to 400 µ mol/L). To the wells of a 96-well plate, there were added 10 µ L each of these solutions of the test compound, [³H]-cAMP diluted with a buffer solution (40 mmol/L Tris-HCl (pH 7.4), containing 10 mmol/L of MgCl₂) and 40 µ L of each human-derived recombinant PDE protein in an amount of 2x10⁻⁶ units (wherein one unit means the amount of each PDE required for decomposing 1 µ mol/L of cAMP, within one minute, at a pH value of 7.5 and a temperature of 30°C) and they were reacted at 30°C for 20 minutes. After they were reacted at 65°C for additional 2 minutes, 25 µ L each of a 1 mg/mL 5' nucleotidase solution (Crotalus atrox venom available from Sigma Company) was added to each well and the reaction was continued at 30°C for 10 minutes. After the completion of the reaction, 200 µ L each of Dowex solution (300 mg/mL Dowex 1x8-400 (available from Sigma Aldrich Company), 33% Ethanol) was added, followed by the oscillation mixing thereof at 4°C for 20 minutes, the addition of 200 µ L each of MicroScint 20 (available from Packard Company) and the measurement using a scintillation counter (Topcount, available from Packard Company). The IC₅₀ value of each test compound was calculated by the use of GraphPad Prism v3. 03 (available from GraphPad Software Company).

In this connection, the results thus obtained are expressed as follows: IC₅₀ value ≧ 100 µ mol/L (NI); 100 µ mol/L> IC₅₀ value ≧ 1 µ mol/L (-); 1 µ mol/L>
IC₅₀ value≧0.1 µ mol/L (+); 0.1 µ mol/L> IC₅₀ value≧0.01 µ mol/L (++); 0.01 µ mol/L> IC₅₀ value (+++).

The results obtained are summarized in the following Table 1.

**Table 1**

| Example No | IC₅₀)(µmol/L) PDE3 | IC₅₀(µmol/L) PDE4 | Example No | IC₅₀(µmol/L) PRE3 | IC₅₀(µmol/L) PDE4 |
|---|---|---|---|---|---|
| 47 | NI | +++ | 54 | - | +++ |
| 48 | - | +++ | 55 | NI | +++ |
| 49 | - | +++ | 56 | NI | +++ |
| 50 | - | +++ | 57 | NI | +++ |
| 51 | NI | +++ | 58 | NI | +++ |
| 52 | NI | +++ | 59 | - | +++ |
| 53 | - | +++ | | | |

### Test Example 2: LPS -induced Acute Pulmonary Inflammation in Rats:

One hour before the inhalation of lipopolysaccharide from E. coli serotype 055:B5 (LPS), each compound was orally administered to each rat in a dose of 3 mg/kg, and then the animal was allowed to inhale an LPS solution (50 mL) over 30 minutes, while the solution was atomized using a nebulizer. After 3 hours from the LPS-inhalation, the rats were euthanized by the administration of a 20% urethane solution (5 mL/rat, i.p.). Broncoalveolar lavage was performed by injecting 5 ml physiological saline, three consecutive times using a 5 mL volume syringe. The operations were carried out twice and these physiological saline samples were recovered as broncoalveolar lavage fluid (BALF). Each of the BALF thus recovered was centrifuged at 1200 rpm and 4°C for 10 minutes (Hirtachi; himac CR 5 DL), the resulting residue was again suspended in 10 mL of 0.1% bovine serum albumin-containing physiological saline, an equivalent volume of Turk's diluting fluid was added to the suspension to thus stain the leukocytes present therein and the total number of leukocytes was determined under the microscopic observation to calculate the rate of inhibition.
As a result of the determination according to the foregoing method, it was found that the compounds prepared in, for instance, Examples 47, 50, 51, 56, 57 and 58 showed an inhibitory effect of not less than 50%.
As has been described above, the compounds represented by the general formula (1) according to the present invention show a PDE-inhibitory activity and the effectiveness thereof has been confirmed in a variety of animal test models.

### Industrial Applicability

As has been described above in detail, according to the present invention, it has been found that a novel isoquinoline derivative and an addition salt thereof show excellent PDE inhibitory activity. Such a compound having a PDE inhibitory activity is useful as an agent for treating angina, heart failure and hypertension; a platelet aggregation inhibitor; an agent for preventing or treating bronchial asthma, chronic obstructive pulmonary diseases (COPD), interstitial pneumonia, interstitial cystitis, allergic conjunctivitis, allergic rhinitis, atopic dermatitis, osteoporosis, osteoarthritis of knee, rheumatoid arthritis, non-alcoholic fatty liver, multiple sclerosis, Crohn's disease, inflammatory colitis, a variety of mental disorders such as Huntington's disease, Alzheimer's disease, dementia, Parkinson's disease, depression and schizophrenia, obesity and metabolic syndromes; as well as an agent for the treatment of the male impotence.

## Claims

1. An isoquinoline derivative represented by the following general formula (1), an optically active derivative thereof or a pharmaceutically acceptable salt or hydrate thereof: 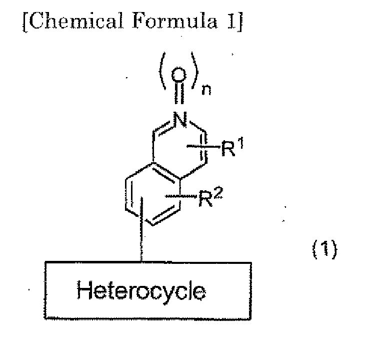 [wherein R¹ and R² may be the same or different and represent a hydrogen atom or a halogen atom; n represents 0 or 1; and Heterocycle represents a heterocyclic group represented by one of the following general formulas: 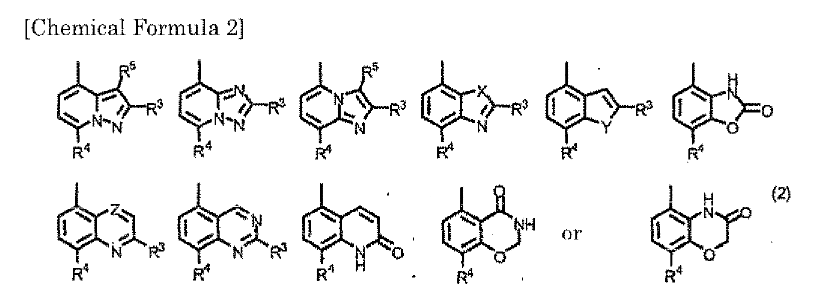 [wherein R³ represents a hydrogen atom, an alkyl group having 1 to 6 carbon atoms, which may be substituted with a halogen atom, or a cycloalkyl group having 3 to 8 carbon atoms; R⁴ represents an alkoxy group having 1 to 6 carbon atoms, an amino group or an alkylamino group having 1 to 6 carbon atoms; R⁵ represents a hydrogen atom or a halogen atom; X represents NH, O or S; Y represents O or S; and Z represents CH or N].

2. The isoquinoline derivative, optically active derivative thereof or pharmaceutically acceptable salt or hydrate thereof of claim 1, wherein the position of the Heterocycle moiety linked to the isoquinoline ring is represented by the following general formula (1a): 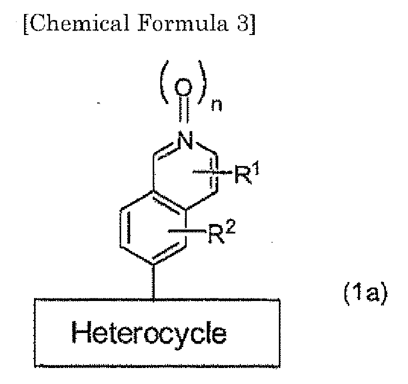 [wherein R¹, R², n and Heterocycle are the same as those defined above].

3. The isoquinoline derivative, optically active derivative thereof or pharmaceutically acceptable salt or hydrate thereof of claim 1, wherein the compound represented by the foregoing general formula (1) is one represented by the following general formula: 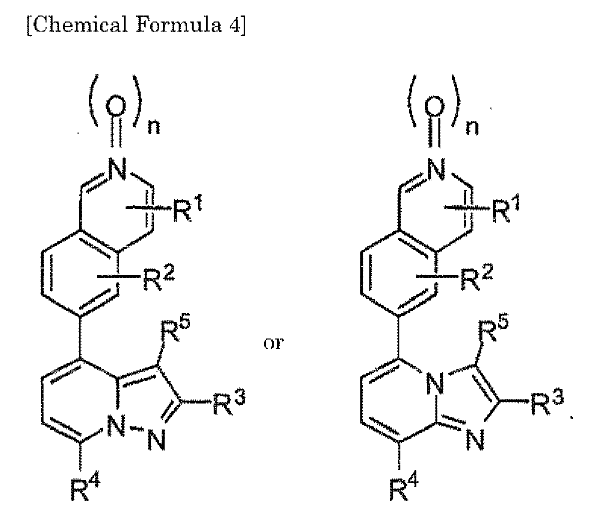 [wherein R¹, R², R³, R⁴, R⁵ and n are the same as those defined above].

4. The isoquinoline derivative, optically active derivative thereof or pharmaceutically acceptable salt or hydrate of any one of claims 1 to 3, wherein the substituent R⁴ of the compound represented by the foregoing general formula (1) is an alkoxy group having 1 to 6 carbon atoms.

5. The isoquinoline derivative, optically active derivative thereof or pharmaceutically acceptable salt or hydrate thereof of any one of claims 1 to 3, wherein the substituent R⁴ of the compound represented by the foregoing general formula (1) is an alkylamino group having 1 to 6 carbon atoms.

6. The isoquinoline derivative, optically active derivative thereof or pharmaceutically acceptable salt or hydrate thereof of claim 1, wherein the compound represented by the foregoing general formula (1) is:
(1) 6-(7-Methoxy-2-trifluoromethylpyrazolo[1,5-a]pyridin-4-yl)isoquinoline;
(2) 5-Chloro-6-(7-methoxy-2-trifluoromethylpyrazolo[1,5-a]pyridin-4-yl) isoquinoline;
(3) 6-(3-Chloro-7-methoxy-2-trifluoromethylpyrazolo[1,5-a]pyridin-4-yl) isoquinoline;
(4) 3-Chloro-4-(isoquinolin-6-yl)-N-methyl-2-trifluoromethylpyrazolo[1,5-a] pyridine-7-amine;
(5) 6-(3-Chloro-8-methoxy-2-trifluoromethylimidazo[1,2-a]pyridin-5-yl) isoquinoline; or
(6) 3-Chloro-5-(isoquinolin-6-yl)-N-methyl-2-trifluoromethylimidazo[1,2-a] pyridine-8-amine.

7. A phosphodiesterase (PDE) inhibitor comprising, as an effective component, the isoquinoline derivative, optically active derivative thereof or pharmaceutically acceptable salt or hydrate thereof of any one of claims 1 to 6.

8. A pharmaceutical composition comprising, as an effective component, the isoquinoline derivative, optically active derivative thereof or pharmaceutically acceptable salt or hydrate thereof of any one of claims 1 to 6.

9. The pharmaceutical agent of claim 8 for preventing or treating angina, heart failure, hypertension, bronchial asthma, chronic obstructive pulmonary diseases (COPD), interstitial pneumonia, interstitial cystitis, allergic conjunctivitis, allergic rhinitis, atopic dermatitis, osteoporosis, rheumatoid arthritis, osteoarthritis of knee, non-alcoholic fatty liver, multiple sclerosis, Crohn's disease, inflammatory colitis, Huntington's disease, Alzheimer's disease, dementia, Parkinson's disease, depression, schizophrenia, obesity and metabolic syndromes.
